# EUROPEAN PATENT SPECIFICATION

(11) **EP 4 593 789 B1**
(45) Date of publication and mention of the grant of the patent: **10.06.2026**
(21) Application number: 23786789.0
(22) Date of filing: 08.09.2023
(51) Int. Cl.: A61K 8/55, A61K 8/73, A61K 8/88, A61Q 19/00

(54) **COMPOSITION COMPRISING HYALURONIC ACID-BASED POLYION COMPLEX AND SUPERABSORBENT POLYMER**
ZUSAMMENSETZUNG MIT POLYIONENKOMPLEX AUF HYALURONSÄUREBASIS UND SUPERABSORBIERENDEM POLYMER
COMPOSITION COMPRENANT UN COMPLEXE POLYIONIQUE À BASE D'ACIDE HYALURONIQUE ET UN POLYMÈRE SUPERABSORBANT

(30) Priority: 27.09.2022 JP 2022153796; 28.10.2022 FR 2211244
(43) Date of publication of application: 06.08.2025
(73) Proprietor: L'OREAL, 75008 Paris (FR); Mitsuda, Shinobu, Kawasaki-shi, Kanagawa 2130012 (JP); Isojima, Tatsushi, Kawasaki-shi, Kanagawa, 213-0012 (JP); Yamamoto, Mariko, Kawasaki-shi, Kanagawa, 213-0012 (JP)
(72) Inventor: MITSUDA, Shinobu, Kawasaki-shi, Kanagawa 2130012 (JP); ISOJIMA, Tatsushi, Kawasaki-shi, Kanagawa 2130012 (JP); YAMAMOTO, Mariko, Kawasaki-shi, Kanagawa 2130012 (JP)
(74) Representative: Ipsilon
(86) International application number: PCT/JP2023/033632
(87) International publication number: WO 2024/070749

(56) References cited:
- WO-A1-2021/125069
- WO-A1-2021/130677
- WO-A1-2022/131351
- FR-A1- 2 984 125
- JP-A- 2022 093 930

## Description

### TECHNICAL FIELD

The present invention relates to a composition including a polyion complex and a superabsorbent polymer, as well as a cosmetic process using the composition.

### BACKGROUND ART

Hyaluronic acid is a predominant glucosaminoglycan found in the skin. Thus, the fibroblasts synthesize predominantly collagens, matrix glycoproteins other than collagens (fibronectin, laminin), proteoglycans and elastin. The keratinocytes, for their part, synthesize predominantly sulfated glycosaminoglycans and hyaluronic acid. Hyaluronic acid is also called hyaluronan (HA).

Hyaluronic acid is present in a free state in the epidermis and in the dermis and is responsible for turgescence of the skin. This polysaccharide can in fact retain a large volume of water, corresponding to up to 1000 times its weight. In this sense, hyaluronic acid plays an important role in increasing the amounts of water bound in the tissue, and also in the mechanical properties of the skin and in wrinkle formation.

Hyaluronic acid has been widely used as a cosmetic ingredient due to its high moisturizing effects.

WO 2021/125069 discloses a polyion complex which may be composed of at least one cationic polymer and at least one anionic polymer, wherein the anionic polymer is selected from hyaluronic acid and derivatives thereof.

### DISCLOSURE OF INVENTION

It has been found that a composition comprising a hyaluronic acid-based polyion complex may become unstable over time, and that the composition may exert relatively limited sebum control effects.

Thus, an objective of the present invention is to provide a stable composition comprising a hyaluronic acid-based polyion complex with enhanced sebum control effects.

The above objective of the present invention can be achieved by a composition, comprising:
(a) at least one cationic polymer;
(b) at least one anionic polymer;
(c) at least one non-polymeric acid having two or more pKa values or salt(s) thereof;
(d) at least one superabsorbent polymer; and
(e) water,
wherein
the (b) anionic polymer is selected from hyaluronic acids and salts thereof, and
the (d) superabsorbent polymer is in the form of particles with a number average particle size of 5 µm to 500 µm, preferably 10 µm to 400 µm, and more preferably 15 µm to 300 µm.

The (a) cationic polymer may have at least one positively chargeable and/or positively charged moiety selected from the group consisting of a primary, secondary or tertiary amino group, a quaternary ammonium group, a guanidine group, a biguanide group, an imidazole group, an imino group, and a pyridyl group.

The (a) cationic polymer may be selected from the group consisting of cyclopolymers of alkyldiallylamine and cyclopolymers of dialkyldiallylammonium such as (co)polydiallyldialkyl ammonium chloride, (co)polyamines such as chitosans and (co)polylysines, cationic (co)polyaminoacids such as collagen, cationic cellulose polymers, and salts thereof.

The amount of the (a) cationic polymer(s) in the composition according to the present invention may be from 0.01% to 15% by weight, preferably from 0.05% to 10% by weight, and more preferably from 0.1% to 5% by weight, relative to the total weight of the composition.

The composition according to the present invention may comprise at least two hyaluronic acids with different molecular weights, preferably a first hyaluronic acid with a molecular weight of 100 kDa or less and a second hyaluronic acid with a molecular weight of 500 kDa or more, and more preferably a first hyaluronic acid with a molecular weight of 50 kDa or less and a second hyaluronic acid with a molecular weight of 1000 kDa or more, as the (b) anionic polymers.

The amount of the (b) anionic polymer(s) in the composition according to the present invention may be from 0.01% to 15% by weight, preferably from 0.05% to 10% by weight, and more preferably from 0.1% to 5% by weight, relative to the total weight of the composition.

The (c) non-polymeric acid having two or more pKa values or salt(s) thereof may be an organic acid or salt(s) thereof, preferably a hydrophilic or water-soluble organic acid or salt(s) thereof, and more preferably phytic acid or salts thereof.

The amount of the (c) non-polymeric acid having two or more pKa values or salt(s) thereof in the composition according to the present invention may be from 0.01% to 15% by weight, preferably from 0.05% to 10% by weight, and more preferably from 0.1% to 5% by weight, relative to the total weight of the composition.

The (d) superabsorbent polymer may be selected from crosslinked (meth)acrylic or (meth)acrylate polymers, starches grafted with acrylic or acrylate polymer(s), and mixtures thereof.

The amount of the (d) superabsorbent polymer in the composition according to the present invention may be from 0.01% to 15% by weight, preferably from 0.05% to 10% by weight, and more preferably from 0.01% to 5% by weight, relative to the total weight of the composition.

The amount of the (e) water in the composition according to the present invention may be from 50% to 95% by weight, preferably from 60% to 90% by weight, and more preferably from 70% to 85% by weight, relative to the total weight of the composition.

The pH of the composition according to the present invention may be from 3 to 9, preferably from 3.3 to 8.0, and more preferably from 3.5 to 8.

The composition according to the present invention may further comprise (f) at least one oil.

The composition according to the present invention may be a cosmetic composition, preferably a skin cosmetic composition, and more preferably a skin care cosmetic composition.

The present invention also relates to a cosmetic process for a keratin substance such as skin, comprising:
applying, to the keratin substance, the composition according to the present invention; and
drying the composition to form a cosmetic film on the keratin substance.

### BEST MODE FOR CARRYING OUT THE INVENTION

After diligent research, the inventors have discovered that it is possible to provide a stable composition comprising a hyaluronic acid-based polyion complex with enhanced sebum control effects.

Thus, the composition according to the present invention comprises:
(a) at least one cationic polymer;
(b) at least one anionic polymer;
(c) at least one non-polymeric acid having two or more pKa values or salt(s) thereof;
(d) at least one superabsorbent polymer; and
(e) water,
wherein
the (b) anionic polymer is selected from hyaluronic acids and salts thereof, and
the (d) superabsorbent polymer is in the form of particles with a number average particle size of 5
µm to 500 µm, preferably 10 µm to 400 µm, and more preferably 15 µm to 300 µm.

The composition according to the present invention is stable for a long period of time. The composition is stable even if it includes oil(s).

The composition according to the present invention can provide excellent sebum control effects. For example, the film formed by the composition according to the present invention can absorb sebum or oil and maintain it in the film even for a long period of time. The sebum control effects can result in optical matte effects on a keratin substance such as skin.

Further, the composition according to the present invention can be applied without forming aggregations which may be in the form of noodles.

Furthermore, the composition according to the present invention can provide cosmetic effects, such as moisturizing effects, based on the hyaluronic acid or salts thereof.

In addition, the composition according to the present invention could be less sticky. For example, the stickiness of the composition according to the present invention including hyaluronic acid as an ingredient forming a polyion complex could be reduced as compared to a composition including hyaluronic acid without forming a polyion complex.

Hereinafter, the composition, process, and the like according to the present invention will be explained in a more detailed manner.

### [Polyion Complex]

The composition according to the present invention includes (a) at least one cationic polymer, (b) at least one anionic polymer, and (c) at least one non-polymeric acid having two or more pKa values or salt(s) thereof, explained below.

The above ingredients (a), (b) and (c) can form a polyion complex. The polyion complex may be in the form of a particle. Two or more different types of particles may be present in combination. Thus, a single type of a particle or a combination of different types of particles may be present in the composition according to the present invention.

If the polyion complex is in the form of a particle, the size of the particle may be from 5 nm to 100 µm, preferably from 100 nm to 50 µm, more preferably from 200 nm to 40 µm, and even more preferably from 500 nm to 30 µm. A particle size less than 1 µm can be measured by a dynamic light scattering method, and a particle size more than 1 µm can be measured by an optical microscope. This particle size may be based on number average diameter.

The amount of the particle(s) in the composition according to the present invention may be 0.01% by weight or more, preferably 0.05% by weight or more, and more preferably 0.1% by weight or more, relative to the total weight of the composition.

The amount of the particle(s) in the composition according to the present invention may be 15% by weight or less, preferably 10% by weight or less, and more preferably 5% by weight or less, relative to the total weight of the composition.

The amount of the particle(s) in the composition according to the present invention may be from 0.01% to 15% by weight, preferably from 0.05% to 10% by weight, and more preferably from 0.1% to 5% by weight, relative to the total weight of the composition.

If the composition according to the present invention includes (f) at least one oil, explained later, a plurality of the particles can be present at the interface between the (e) water and the (f) oil. Thus, the particles can stabilize an emulsion. For example, if the (e) water constitutes a continuous phase and the (f) oil constitutes dispersed phases, the particles can form an O/W emulsion which may be similar to a so-called Pickering emulsion.

Alternatively, a plurality of the particles can form a capsule having a hollow portion. The (f) oil can be present in the hollow portion. In other words, the (f) oil can be incorporated into the capsule. The wall of the capsule may be composed of a continuous layer or film formed from the particles. While not wishing to be bound by theory, it is believed that the particles can be re-organized at the interface of the (e) water and the (f) oil to spontaneously form a capsule having a hollow portion to include the (f) oil. For example, a continuous phase constituted with the (e) water and dispersed phases constituted with the (f) oil in the capsule can form an O/W emulsion which may also be similar to a so-called Pickering emulsion.

The above would mean that the particle itself is amphiphilic. The particle itself is insoluble in oil or water.

### (Cationic Polymer)

The composition according to the present invention includes (a) at least one cationic polymer. Two or more different types of cationic polymers may be used in combination. Thus, a single type of a cationic polymer or a combination of different types of cationic polymers may be used.

The (a) cationic polymer has a positive charge density. The charge density of the (a) cationic polymer may be from 0.01 meq/g to 20 meq/g, preferably from 0.05 to15 meq/g, and more preferably from 0.1 to 10 meq/g.

It may be preferable that the molecular weight of the (a) cationic polymer be 500 or more, preferably 1000 or more, more preferably 2000 or more, and even more preferably 3000 or more. Unless otherwise defined in the descriptions, "molecular weight" means a number average molecular weight.

The (a) cationic polymer may have at least one positively chargeable and/or positively charged moiety selected from the group consisting of a primary, secondary or tertiary amino group, a quaternary ammonium group, a guanidine group, a biguanide group, an imidazole group, an imino group, and a pyridyl group. The term "(primary) amino group" here means an -NH₂ group.

The (a) cationic polymer may be a homopolymer or a copolymer. The term "copolymer" is understood to mean both copolymers obtained from two kinds of monomers and those obtained from more than two kinds of monomers, such as terpolymers obtained from three kinds of monomers.

The (a) cationic polymer may be selected from natural and synthetic cationic polymers. Non-limiting examples of the (a) cationic polymer are as follows.
(1) Homopolymers and copolymers derived from acrylic or methacrylic esters and amides and comprising at least one unit chosen from units of the following formulas: 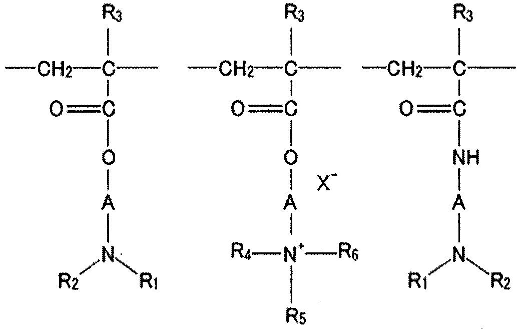 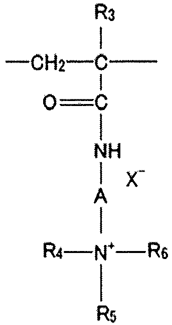 wherein:
   R₁ and R₂, which may be identical or different, are chosen from hydrogen and alkyl groups comprising from 1 to 6 carbon atoms, for instance, methyl and ethyl groups;
   R₃, which may be identical or different, is chosen from hydrogen and CH₃;
   the symbols A, which may be identical or different, are chosen from linear or branched alkyl groups comprising from 1 to 6 carbon atoms, for example, from 2 to 3 carbon atoms, and hydroxyalkyl groups comprising from 1 to 4 carbon atoms;
   R₄, R₅, and R₆, which may be identical or different, are chosen from alkyl groups comprising from 1 to 18 carbon atoms and benzyl groups, and in at least one embodiment, alkyl groups comprising from 1 to 6 carbon atoms; and
   X is an anion derived from an inorganic or organic acid, such as methosulphate anions and halides, for instance chloride and bromide.

The copolymers of family (1) above may also comprise at least one unit derived from comonomers which may be chosen from acrylamides, methacrylamides, diacetone acrylamides, acrylamides and methacrylamides substituted on the nitrogen atom with (C₁-C₄) lower alkyl groups, groups derived from acrylic or methacrylic acids and esters thereof, vinyllactams such as vinylpyrrolidone and vinylcaprolactam, and vinyl esters.

Examples of copolymers of family (1) include, but are not limited to:
copolymers of acrylamide and of dimethylaminoethyl methacrylate quaternized with dimethyl sulphate or with a dimethyl halide,
copolymers of acrylamide and of methacryloyloxyethyltrimethylammonium chloride described, for example, in European Patent Application No. 0 080 976,
copolymers of acrylamide and of methacryloyloxyethyltrimethylammonium methosulphate,
quaternized or nonquaternized vinylpyrrolidone/dialkylaminoalkyl acrylate or methacrylate copolymers, described, for example, in French Patent Nos. 2 077 143 and 2 393 573, dimethylaminoethyl methacrylate/vinylcaprolactam/vinylpyrrolidone terpolymers, vinylpyrrolidone/methacrylamidopropyldimethylamine copolymers, quaternized vinylpyrrolidone/dimethylaminopropylmethacrylamide copolymers, and
crosslinked methacryloyloxy(C₁-C₄)alkyltri(C₁-C₄)alkylammonium salt polymers such as the polymers obtained by homopolymerization of dimethylaminoethyl methacrylate quaternized with methyl chloride, or by copolymerization of acrylamide with dimethylaminoethyl methacrylate quaternized with methyl chloride, the homopolymerization or copolymerization being followed by crosslinking with a compound containing an olefinic unsaturation, for example, methylenebisacrylamide.
(2) Cationic cellulose polymers such as cellulose ether derivatives comprising one or more quaternary ammonium groups described, for example, in French Patent No. 1 492 597, such as the polymers sold under the names "JR" (JR 400, JR 125, JR 30M) or "LR" (LR 400, LR 30M) by the company Union Carbide Corporation. These polymers are also defined in the CTFA dictionary as quaternary ammoniums of hydroxyethylcellulose that have reacted with an epoxide substituted with a trimethylammonium group.

It is preferable that the cationic cellulose polymers have at least one quaternary ammonium group, preferably a quaternary trialkyl ammonium group, and more preferably a quaternary trimethyl ammonium group.

The quaternary ammonium group may be present in a quaternary ammonium group-containing group which may be represented by the following chemical formula (I): 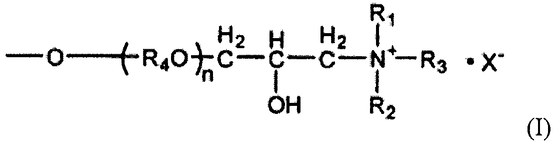 wherein
each of R₁ and R₂ denotes a C₁-C₃ alkyl group, preferably a methyl or ethyl group, and more preferably a methyl group,
R₃ denotes a C₁-C₂₄ alkyl group, preferably a methyl or ethyl group, and more preferably methyl group,
X- denotes an anion, preferably a halide, and more preferably a chloride,
n denotes an integer from 0-30, preferably 0-10, and more preferably 0, and
R₄ denotes a C₁-C₄ alkylene group, preferably an ethylene or propylene group.

The leftmost ether bond (-O-) in the above chemical formula (I) can attach to the sugar ring of the polysaccharide.

It is preferable that the quaternary ammonium group-containing group be -O-CH₂-CH(OH)-CH₂-N⁺(CH₃)₃.

(3) Cationic cellulose polymers such as cellulose copolymers and cellulose derivatives grafted with a water-soluble monomer of quaternary ammonium, and described, for example, in U.S. Pat. No. 4,131,576, such as hydroxyalkylcelluloses, for instance, hydroxymethyl-, hydroxyethyl-, and hydroxypropylcelluloses grafted, for example, with a salt chosen from methacryloylethyltrimethylammonium, methacrylamidopropyltrimethylammonium, and dimethyldiallylammonium salts.

Commercial products corresponding to these polymers include, for example, the products sold under the name "Celquat^{®} L 200" and "Celquat^{®} H 100" by the company National Starch.

(4) Non-cellulose-based cationic polysaccharides described in U.S. Pat. Nos. 3,589,578 and 4,031,307, such as guar gums comprising cationic trialkylammonium groups, cationic hyaluronic acid, and dextran hydroxypropyl trimonium chloride. Guar gums modified with a salt, for example the chloride, of 2,3-epoxypropyltrimethylammonium (guar hydroxypropyltrimonium chloride) may also be used.

Such products are sold, for instance, under the trade names JAGUAR^{®} C13 S, JAGUAR^{®} C15, JAGUAR^{®} C17, and JAGUAR^{®} C162 by the company MEYHALL.

(5) Polymers comprising piperazinyl units and divalent alkylene or hydroxyalkylene groups comprising straight or branched chains, optionally interrupted with at least one entity chosen from oxygen, sulphur, nitrogen, aromatic rings, and heterocyclic rings, and also the oxidation and/or quaternization products of these polymers. Such polymers are described, for example, in French Patent Nos. 2 162 025 and 2 280 361.

(6) Water-soluble polyamino amides prepared, for example, by polycondensation of an acidic compound with a polyamine; these polyamino amides possibly being crosslinked with an entity chosen from epihalohydrins; diepoxides; dianhydrides; unsaturated dianhydrides; bisunsaturated derivatives; bishalohydrins; bisazetidiniums; bishaloacyidiamines; bisalkyl halides; oligomers resulting from the reaction of a difunctional compound which is reactive with an entity chosen from bishalohydrins, bisazetidiniums, bishaloacyldiamines, bisalkyl halides, epihalohydrins, diepoxides, and bisunsaturated derivatives; the crosslinking agent being used in an amount ranging from 0.025 to 0.35 mol per amine group of the polyamino amide; these polyamino amides optionally being alkylated or, if they comprise at least one tertiary amine function, they may be quaternized. Such polymers are described, for example, in French Patent Nos. 2 252 840 and 2 368 508.

(7) Polyamino amide derivatives resulting from the condensation of polyalkylene polyamines with polycarboxylic acids, followed by alkylation with difunctional agents, for example, adipic acid/dialkylaminohydroxyalkyldialkylenetriamine polymers in which the alkyl group comprises from 1 to 4 carbon atoms, such as methyl, ethyl, and propyl groups, and the alkylene group comprises from 1 to 4 carbon atoms, such as an ethylene group. Such polymers are described, for instance, in French Patent No. 1 583 363. In at least one embodiment, these derivatives may be chosen from adipic acid/dimethylaminohydroxypropyldiethylenetriamine polymers.

(8) Polymers obtained by reaction of a polyalkylene polyamine comprising two primary amine groups and at least one secondary amine group, with a dicarboxylic acid chosen from diglycolic acid and saturated aliphatic dicarboxylic acids comprising from 3 to 8 carbon atoms. The molar ratio of the polyalkylene polyamine to the dicarboxylic acid may range from 0.8:1 to 1.4:1; the polyamino amide resulting therefrom being reacted with epichlorohydrin in a molar ratio of epichlorohydrin relative to the secondary amine group of the polyamino amide ranging from 0.5:1 to 1.8:1. Such polymers are described, for example, in U.S. Pat. Nos. 3,227,615 and 2,961,347.

(9) Cyclopolymers of alkyldiallylamine and cyclopolymers of dialkyldiallyl-ammonium, such as homopolymers and copolymers comprising, as the main constituent of the chain, at least one unit chosen from units of formulas (Ia) and (Ib): 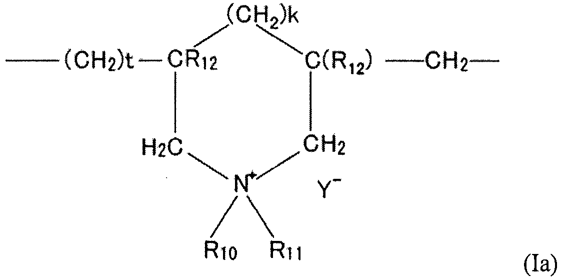 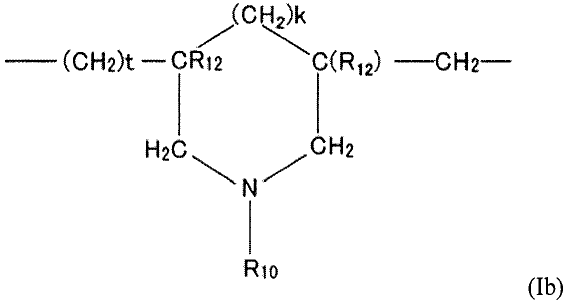 wherein:
k and t, which may be identical or different, are equal to 0 or 1, the sum k+t being equal to 1;
R₁₂ is chosen from hydrogen and methyl groups;
R₁₀ and R₁₁, which may be identical or different, are chosen from alkyl groups comprising from 1 to 6 carbon atoms, hydroxyalkyl groups in which the alkyl group comprises, for example, from 1 to 5 carbon atoms, and lower (C₁-C₄)amidoalkyl groups, or R₁₀ and R₁₁ may form, together with the nitrogen atom to which they are attached, heterocyclic groups such as piperidinyl and morpholinyl; and
Y' is an anion such as bromide, chloride, acetate, borate, citrate, tartrate, bisulphate, bisulphite, sulphate, and phosphate. These polymers are described, for example, in French
Patent No. 2 080 759 and in its Certificate of Addition 2 190 406.

In one embodiment, R₁₀ and R₁₁, which may be identical or different, are chosen from alkyl groups comprising from 1 to 4 carbon atoms.

Examples of such polymers include, but are not limited to, (co)polydiallyldialkyl ammonium chloride such as the dimethyidiallylammonium chloride homopolymer sold under the name "MERQUAT^{®} 100" by the company CALGON (and its homologues of low weight-average molecular mass) and the copolymers of diallyldimethylammonium chloride and of acrylamide sold under the name "MERQUAT^{®} 550".

Quaternary diammonium polymers comprising at least one repeating unit of formula (II): 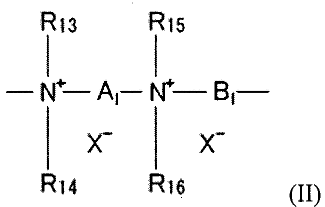 wherein:
R₁₃, R₁₄, R₁₅, and R₁₆, which may be identical or different, are chosen from aliphatic, alicyclic, and arylaliphatic groups comprising from 1 to 20 carbon atoms and lower hydroxyalkyl aliphatic groups, or alternatively R₁₃, R₁₄, R₁₅, and R₁₆ may form, together or separately, with the nitrogen atoms to which they are attached, heterocycles optionally comprising a second heteroatom other than nitrogen, or alternatively R₁₃, R₁₄, R₁₅, and R₁₆, which may be identical or different, are chosen from linear or branched C₁-C₆ alkyl groups substituted with at least one group chosen from nitrile groups, ester groups, acyl groups, amide groups, -CO-O-R₁₇-E groups, and -CO-NH-R₁₇-E groups, wherein R₁₇ is an alkylene group and E is a quaternary ammonium group;
A₁ and B₁, which may be identical or different, are chosen from polymethylene groups comprising from 2 to 20 carbon atoms, which may be linear or branched, saturated or unsaturated, and which may comprise, linked or intercalated in the main chain, at least one entity chosen from aromatic rings, oxygen, sulphur, sulphoxide groups, sulphone groups, disulphide groups, amino groups, alkylamino groups, hydroxyl groups, quaternary ammonium groups, ureido groups, amide groups, and ester groups, and
X⁻ is an anion derived from an inorganic or organic acid;
A₁, R₁₃, and R₁₅ may form, together with the two nitrogen atoms to which they are attached, a piperazine ring;
if A₁ is chosen from linear or branched, saturated or unsaturated alkylene or hydroxyalkylene groups, B₁ may be chosen from:

   -(CH₂)ₙ-CO-E'-OC-(CH₂)ₙ-
wherein E' is chosen from:
   a) glycol residues of formula -O-Z-O-, wherein Z is chosen from linear or branched hydrocarbon-based groups and groups of the following formulas:

      -(CH₂-CH₂-O)ₓ-CH₂-CH₂-

      -[CH₂-CH(CH₃)-O]_{y}-CH₂-CH(CH₃)-

      wherein x and y, which may be identical or different, are chosen from integers ranging from 1 to 4, which represent a defined and unique degree of polymerization, and numbers ranging from 1 to 4, which represent an average degree of polymerization;
   b) bis-secondary diamine residue such as piperazine derivatives;
   c) bis-primary diamine residues of formula -NH-Y-NH-, wherein Y is chosen from linear or branched hydrocarbon-based groups and the divalent group -CH₂-CH₂-S-S-CH₂-CH₂-; and
   d) ureylene groups of formula -NH-CO-NH-.

In at least one embodiment, X⁻ is an anion such as chloride or bromide.

Polymers of this type are described, for example, in French Patent Nos. 2 320 330; 2 270 846; 2 316 271; 2 336 434; and 2 413 907 and U.S. Pat. Nos. 2,273,780; 2,375,853; 2,388,614; 2,454,547; 3,206,462; 2,261,002; 2,271,378; 3,874,870; 4,001,432; 3,929,990; 3,966,904; 4,005,193; 4,025,617; 4,025,627; 4,025,653; 4,026,945; and 4,027,020.

Non-limiting examples of such polymers include those comprising at least one repeating unit of formula (III): 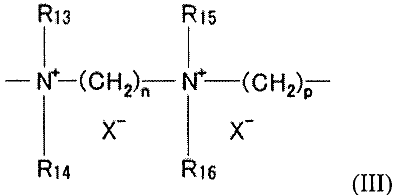 wherein R₁₃, R₁₄, R₁₅, and R₁₆, which may be identical or different, are chosen from alkyl and hydroxyalkyl groups comprising from 1 to 4 carbon atoms, n and p, which may be identical or different, are integers ranging from 2 to 20, and X⁻ is an anion derived from an inorganic or organic acid.

(11) Polyquaternary ammonium polymers comprising units of formula (IV): 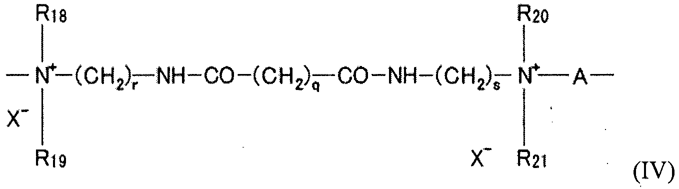 wherein:
R₁₈, R₁₉, R₂₀, and R₂₁, which may be identical or different, are chosen from hydrogen, methyl groups, ethyl groups, propyl groups, β-hydroxyethyl groups, β-hydroxypropyl
groups, -CH₂CH₂(OCH₂CH₂)ₚOH groups, wherein p is chosen from integers ranging from 0 to 6, with the proviso that R₁₈, R₁₉, R₂₀, and R₂₁ are not simultaneously hydrogen,
r and s, which may be identical or different, are chosen from integers ranging from 1 to 6,
q is chosen from integers ranging from 0 to 34,
X⁻ is an anion such as a halide, and
A is chosen from radicals of dihalides and -CH₂-CH₂-O-CH₂-CH₂-.

Such compounds are described, for instance, in European Patent Application No. 0 122 324.

(12) Quaternary polymers of vinylpyrrolidone and of vinylimidazole.

Other examples of suitable cationic polymers include, but are not limited to, cationic proteins and cationic protein hydrolysates, polyalkyleneimines, such as polyethyleneimines, polymers comprising units chosen from vinylpyridine and vinylpyridinium units, condensates of polyamines and of epichlorohydrin, quaternary polyureylenes, and chitin derivatives.

According to one embodiment of the present invention, the (a) at least one cationic polymer is chosen from cellulose ether derivatives comprising quaternary ammonium groups, such as the products sold under the name "JR 400" by the company UNION CARBIDE CORPORATION, cationic cyclopolymers, for instance, the homo-polymers and copolymers of dimethyldiallylammonium chloride sold under the names MERQUAT^{®} 100, MERQUAT^{®} 550, and MERQUAT^{®} S by the company CALGON, guar gums modified with a 2,3-epoxypropyltrimethylammonium salt, and quaternary polymers of vinylpyrrolidone and of vinylimidazole.

### (13) Polyamines

As the (a) cationic polymer, it is also possible to use (co)polyamines, which may be homopolymers or copolymers, with a plurality of amino groups. The amino group may be a primary, secondary, tertiary or quaternary amino group. The amino group may be present in a polymer backbone or a pendent group, if present, of the (co)polyamines.

As an example of the (co)polyamines, mention may be made of chitosan, (co)polyallylamines, (co)polyvinylamines, (co)polyanilines, (co)polyvinylimidazoles,
(co)polydimethylaminoethylenemethacrylates, (co)polyvinylpyridines such as (co)poly-1-methyl-2-vinylpyridines, (co)polyimines such as (co) polyethyleneimines, (co)polypyridines such as (co)poly(quaternary pyridines), (co)polybiguanides such as (co)polyaminopropyl biguanides, (co)polylysines, (co)polyornithines, (co)polyarginines, (co)polyhistidines, aminodextrans, aminocelluloses, amino(co)polyvinylacetals, and salts thereof.

As the (co)polyamines, it is preferable to use (co)polylysines. Polylysine is well known. Polylysine can be a natural homopolymer of L-lysine that can be produced by bacterial fermentation. For example, polylysine can be ε-Poly-L-lysine, typically used as a natural preservative in food products. Polylysine is a polyelectrolyte which is soluble in polar solvents such as water, propylene glycol and glycerol. Polylysine is commercially available in various forms, such as poly D-lysine and poly L-lysine. Polylysine can be in salt and/or solution form.

### (14) Cationic Polyaminoacids

As the (a) cationic polymer, it may be possible use cationic polyaminoacids, which may be cationic homopolymers or copolymers, with a plurality of amino groups and carboxyl groups. The amino group may be a primary, secondary, tertiary or quaternary amino group. The amino group may be present in a polymer backbone or a pendent group, if present, of the cationic polyaminoacids. The carboxyl group may be present in a pendent group, if present, of the cationic polyaminoacids.

As examples of the cationic polyaminoacids, mention may be made of cationized collagen, cationized gelatin, steardimonium hydroxypropyl hydrolyzed wheat protein, cocodimonium hydroxypropyl hydrolyzed wheat protein, hydroxypropyltrimonium hydrolyzed conchiolin protein, steardimonium hydroxypropyl hydrolyzed soy protein, hydroxypropyltrimonium hydrolyzed soy protein, cocodimonium hydroxypropyl hydrolyzed soy protein, and the like.

The following descriptions relate to preferable embodiments of the (a) cationic polymer.

It may be preferable that the (a) cationic polymer be selected from cationic starches.

As examples of the cationic starches, mention may be made of starches modified with a 2,3-epoxypropyltrimethylammonium salt (e.g. chloride), such as the product known as starch hydroxypropyltrimonium chloride according to the INCl nomenclature and sold under the name SENSOMER Cl-50 from Ondeo or Pencare^{™} DP 1015 from Ingredion.

It may also be preferable that the (a) cationic polymer be selected from cationic gums.

The gums may be, for example, selected from the group consisting of cassia gum, karaya gum, konjac gum, gum tragacanth, tara gum, acacia gum and gum arabic.

Examples of cationic gums include cationic polygalactomannan derivatives such as guar gum derivatives and cassia gum derivatives, e.g., CTFA: Guar Hydroxypropyltrimonium Chloride, Hydroxypropyl Guar Hydroxypropyltrimonium Chloride, and Cassia Hydroxypropyltrimonium Chloride. Guar hydroxypropyltrimonium chloride is commercially available under the Jaguar^{™} trade name series from Rhodia Inc. and the N-Hance trade name series from Ashland Inc. Cassia Hydroxypropyltrimonium Chloride is commercially available under the Sensomer^{™} CT-250 and Sensomer^{™} CT-400 trademarks from Lubrizol Advanced Materials, Inc. or the ClearHance^{™} from Ashland Inc.

It may be preferable that the (a) cationic polymer be selected from the group consisting of cyclopolymers of alkyldiallylamine and cyclopolymers of dialkyldiallylammonium such as (co)polydiallyldialkyl ammonium chloride, (co)polyamines such as chitosans and (co)polylysines, cationic (co)polyaminoacids such as cationized collagen, cationic cellulose polymers, and salts thereof.

It may be more preferable that the (a) cationic polymer be selected from the group consisting of polylysines such as poly-α-lysine and poly-ε-lysine, polyquaternium-4, polyquaternium-10, polyquaternium-24, polyquaternium-67, starch hydroxypropyl trimonium chloride, cassia hydroxypropyltrimonium chloride, chitosan, and a mixture thereof.

The amount of the (a) cationic polymer(s) in the composition according to the present invention may be 0.01% by weight or more, preferably 0.05% by weight or more, and more preferably 0.1% by weight or more, relative to the total weight of the composition.

The amount of the (a) cationic polymer(s) in the composition according to the present invention may be 15% by weight or less, preferably 10% by weight or less, and more preferably 5% by weight or less, relative to the total weight of the composition.

The amount of the (a) cationic polymer(s) in the composition according to the present invention may be from 0.01% to 15% by weight, preferably from 0.05% to 10% by weight, and more preferably from 0.1% to 5% by weight, relative to the total weight of the composition.

### (Anionic Polymer)

The composition according to the present invention includes (b) at least one anionic polymer. Two or more different types of anionic polymers may be used in combination. Thus, a single type of an anionic polymer or a combination of different types of anionic polymers may be used.

The (b) anionic polymer has a positive charge density. The charge density of the (b) anionic polymer may be from 0.1 meq/g to 20 meq/g, preferably from 1 to15 meq/g, and more preferably from 4 to 10 meq/g if the (b) anionic polymer is a synthetic anionic polymer, and the average substitution degree of the (b) anionic polymer may be from 0.1 to 3.0, preferably from 0.2 to 2.7, and more preferably from 0.3 to 2.5 if the (b) anionic polymer is a natural anionic polymer.

According to the present invention, the (b) anionic polymer is selected from hyaluronic acids and salts thereof.

Hyaluronic acid can be represented by the following chemical formula.

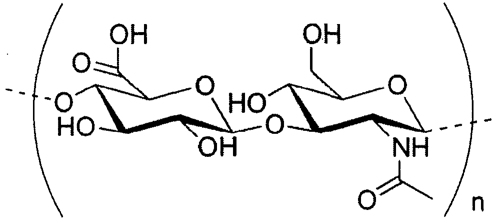

In the context of the present invention, the term "hyaluronic acid" covers in particular the basic unit of hyaluronic acid of formula:

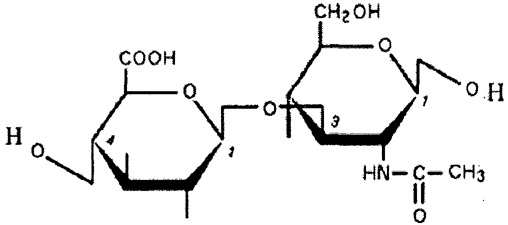

It is the smallest fraction of hyaluronic acid comprising a disaccharide dimer, namely D-glucuronic acid and N-acetylglucosamine.

The term "hyaluronic acid and salts thereof" also comprises, in the context of the present invention, a linear polymer comprising the polymeric unit described above, linked together in the chain via alternating β(1,4) and β(1,3) glycosidic linkages, having a molecular weight (MW) that can range between 380 and 13 000 000 daltons. This molecular weight depends in large part on the source from which the hyaluronic acid is obtained and/or on the preparation methods.

As the salts, mention may be made of alkaline metal salts such as sodium salts and potassium salts, alkaline earth metal salts such as magnesium salts, ammonium salts, and mixtures thereof.

In the natural state, hyaluronic acid is present in pericellular gels, in the base substance of the connective tissues of vertebrate organs such as the dermis and epithelial tissues, and in particular in the epidermis, in the synovial fluid of the joints, in the vitreous humor, in the human umbilical cord and in the crista galli apophysis.

Thus, the term "hyaluronic acid and salts thereof" comprises all the fractions or subunits of hyaluronic acid having a molecular weight in particular within the molecular weight range mentioned above.

In the context of the present invention, hyaluronic acid fractions which do not have an inflammatory activity are preferably used.

By way of illustration of the various hyaluronic acid fractions, reference may be made to the document "Hyaluronan fragments: an information-rich system", R. Stern et al., European Journal of Cell Biology 58 (2006) 699-715, which reviews the listed biological activities of hyaluronic acid according to its molecular weight.

According to a preferred embodiment of the present invention, the hyaluronic acid fractions suitable for the use covered by the present invention have a molecular weight of between 2 000 and 5 000 000 Da.

The hyaluronic acid fractions that may be suitable for the use covered by the present invention may have a molecular weight of between 400 000 and 5 000 000 Da. In this case, the term used is high-molecular-weight hyaluronic acid.

Alternatively, the hyaluronic acid fractions that may also be suitable for the use covered by the present invention may have a molecular weight of between 200 000 and 400 000 Da. In this case, the term used is intermediate-molecular-weight hyaluronic acid.

Alternatively again, the hyaluronic acid fractions that may also be suitable for the use covered by the present invention may have a molecular weight of less than 200 000 Da. In this case, the term used is low-molecular-weight hyaluronic acid.

Hyaluronic acid may in particular be hyaluronic acid supplied by the company Hyactive under the trade name CPN (MW: 10 to 150 kDa), by the company Soliance under the trade name Cristalhyal (MW: 1.1.times.10⁶), by the company Bioland under the name Nutra HA

(MW: 820 000 Da), by the company Bioland under the name Nutra AF (MW: 69 000 Da), by the company Bioland under the name Oligo HA (MW: 6100 Da) or else by the company Vam Farmacos Metica under the name D Factor (MW: 380 Da).

It is preferable that the composition according to the present invention comprises at least two hyaluronic acids with different molecular weights, as the (b) anionic polymers.

It may be preferable that the two hyaluronic acids be a combination of the high-molecular-weight hyaluronic acid and the medium-molecular weight hyaluronic acid, a combination of the high-molecular-weight hyaluronic acid and the low-molecular weight hyaluronic acid, or a combination of the intermediate-molecular-weight hyaluronic acid and the low-molecular weight hyaluronic acid.

It may be preferable that the composition according to the present invention comprises a first hyaluronic acid with a molecular weight of 100 kDa or less and a second hyaluronic acid with a molecular weight of 500 kDa or more, and more preferably a first hyaluronic acid with a molecular weight of 50 kDa or less and a second hyaluronic acid with a molecular weight of 1000 kDa or more.

The weight ratio of the amount of the first hyaluronic acid:the amount of the second hyaluronic acid may be 90:10 to 10:90, preferably 80:20 to 20:80, more preferably 70:30 to 30:70, and even more preferably 60:40 to 40:60.

By using a combination of the at least two hyaluronic acids, with different molecular weights, the texture of the composition according to the present invention can be improved, and the risk of formation of noodles can be further reduced.

The amount of the (b) anionic polymer(s) in the composition according to the present invention may be 0.01% by weight or more, preferably 0.05% by weight or more, and more preferably 0.1% by weight or more, relative to the total weight of the composition.

The amount of the (b) anionic polymer(s) in the composition according to the present invention may be 15% by weight or less, preferably 10% by weight or less, and more preferably 5% by weight or less, relative to the total weight of the composition.

The amount of the (b) anionic polymer(s) in the composition according to the present invention may be from 0.01% to 15% by weight, preferably from 0.05% to 10% by weight, and more preferably from 0.1% to 5% by weight, relative to the total weight of the composition.

### (Non-Polymeric Acid Having Two or More Acid Dissociation Constants)

The composition according to the present invention includes (c) at least one non-polymeric acid having two or more pKa values or salt(s) thereof, i.e., at least one non-polymeric acid having two or more acid dissociation constants or salt(s) thereof. The pKa value (acid dissociation constant) is well known to those skilled in the art, and should be determined at a constant temperature such as 25°C.

The (c) non-polymeric acid having two or more pKa values or salt(s) thereof can be included in the polyion complex which may be in the form of a particle. The non-polymeric acid having two or more pKa values can function as a crosslinker for the (a) cationic polymer, or the (a) cationic polymer and the (b) anionic polymer.

The term "non-polymeric" here means that the acid is not obtained by polymerizing two or more monomers. Therefore, the non-polymeric acid does not correspond to an acid obtained by polymerizing two or more monomers such as polycarboxylic acid.

It is preferable that the molecular weight of the (c) non-polymeric acid having two or more pKa values or salt(s) thereof is 1000 or less, preferably 800 or less, and more preferably 700 or less.

There is no limit to the type of the (c) non-polymeric acid having two or more pKa values or salt(s) thereof. Two or more different types of non-polymeric acids having two or more pKa values or salts thereof may be used in combination. Thus, a single type of a non-polymeric acid having two or more pKa values or a salt thereof or a combination of different types of non-polymeric acids having two or more pKa values or salts thereof may be used.

The term "salt" here means a salt formed by addition of suitable base(s) to the non-polymeric acid having two or more pKa values, which may be obtained from a reaction with the non-polymeric acid having two or more pKa values with the base(s) according to methods known to those skilled in the art. As the salt, mention may be made of metal salts, for example salts with alkaline metal such as Na and K, and salts with alkaline earth metal such as Mg and Ca, and ammonium salts.

The (c) non-polymeric acid having two or more pKa values or salt(s) thereof may be an organic acid or salt(s) thereof, and preferably a hydrophilic or water-soluble organic acid or salt(s) thereof.

The non-polymeric acid having two or more pKa values may have at least two acid groups selected from the group consisting of a carboxylic group, a sulfuric group, a sulfonic group, a phosphoric group, a phosphonic group, a phenolic hydroxyl group, and a mixture thereof.

The non-polymeric acid having two or more pKa values may be a non-polymeric polyvalent acid.

The non-polymeric acid having two or more pKa values may be selected from the group consisting of dicarboxylic acids, disulfonic acids, and diphosphoric acids, and a mixture thereof.

The (c) non-polymeric acid having two or more pKa values or salt(s) thereof may be selected from the group consisting of oxalic acid, malonic acid, succinic acid, glutaric acid, adipic acid, pimelic acid, suberic acid, azelaic acid, sebacic acid, fumaric acid, maleic acid, malic acid, citric acid, aconitic acid, oxaloacetic acid, tartaric acid, and salts thereof; aspartic acid, glutamic acid, and salts thereof; terephthalylidene dicamphor sulfonic acid or salts thereof (Mexoryl SX), Benzophenone-9; phytic acid, and salts thereof; Red 2 (Amaranth), Red 102 (New Coccine), Yellow 5 (Tartrazine), Yellow 6 (Sunset Yellow FCF), Green 3 (Fast Green FCF), Blue 1 (Brilliant Blue FCF), Blue 2 (Indigo Carmine), Red 201 (Lithol Rubine B), Red 202 (Lithol Rubine BCA), Red 204 (Lake Red CBA), Red 206 (Lithol Red CA), Red 207 (Lithol Red BA), Red 208 (Lithol Red SR), Red 219 (Brilliant Lake Red R), Red 220 (Deep Maroon), Red 227 (Fast Acid Magenta), Yellow 203 (Quinoline Yellow WS), Green 201 (Alizanine Cyanine Green F), Green 204 (Pyranine Conc), Green 205 (Light Green SF Yellowish), Blue 203 (Patent Blue CA), Blue 205 (Alfazurine FG), Red 401 (Violamine R), Red 405 (Permanent Re F5R), Red 502 (Ponceau 3R), Red 503 (Ponceau R), Red 504 (Ponceau SX), Green 401 (Naphtol Green B), Green 402 (Guinea Green B), and Black 401 (Naphtol Blue Black); folic acid, ascorbic acid, erythorbic acid, and salts thereof; cystine and salts thereof; EDTA and salts thereof; glycyrrhizin and salts thereof; and a mixture thereof.

It may be preferable that the (c) non-polymeric acid having two or more pKa values or salt(s) thereof be selected from the group consisting of terephthalylidene dicamphor sulfonic acid and salts thereof (Mexoryl SX), Yellow 6 (Sunset Yellow FCF), ascorbic acid, phytic acid and salts thereof, and a mixture thereof.

The amount of the (c) non-polymeric acid having two or more pKa values or salt(s) thereof in the composition according to the present invention may be 0.01% by weight or more, preferably 0.05% by weight or more, and more preferably 0.1% by weight or more, relative to the total weight of the composition.

The amount of the (c) non-polymeric acid having two or more pKa values or salt(s) thereof in the composition according to the present invention may be 15% by weight or less, preferably 10% by weight or less, and more preferably 5% by weight or less, relative to the total weight of the composition.

The amount of the (c) non-polymeric acid having two or more pKa values or salt(s) thereof in the composition according to the present invention may be from 0.01% to 15% by weight, preferably from 0.05% to 10% by weight, and more preferably from 0.1% to 5% by weight, relative to the total weight of the composition.

### [Superabsorbent Polymer]

The composition according to the present invention includes (d) at least one superabsorbent polymer. Two or more different types of superabsorbent polymers may be used in combination. Thus, a single type of a superabsorbent polymer or a combination of different types of superabsorbent polymers may be used.

The term "superabsorbent polymer" is understood to mean a polymer which is capable, in its dry state, of spontaneously absorbing at least 20 times its own weight of aqueous fluid, in particular of water, and especially of distilled water. Such superabsorbent polymers are described in the work "Absorbent Polymer Technology, Studies in Polymer Science 8" by L. Brannon-Pappas and R. Harland, published by Elsevier, 1990.

These polymers have a high capacity for absorbing and retaining water and aqueous fluids. After absorption of the aqueous liquid, the particles of the polymer thus impregnated with aqueous fluid remain insoluble in the aqueous fluid and thus retain their separated particulate state.

The (d) superabsorbent polymer can have a water-absorbing capacity ranging from 20 to 2000 times its own weight (i.e., 20 g to 2000 g of water absorbed per gram of absorbent polymer), preferably from 30 to 1500 times and better still ranging from 50 to 1000 times. These water-absorbing characteristics are defined at standard temperature (25° C.) and pressure (760 mm Hg, i.e. 100 000 Pa) conditions and for distilled water.

The value of the water-absorbing capacity of a polymer can be determined by dispersing 0.5 g of polymer(s) in 150 g of a water solution, by waiting 20 minutes, by filtering the nonabsorbed solution through a 150 µm filter for 20 minutes and by weighing the nonabsorbed water.

The (d) superabsorbent polymer used in the composition of the present invention is in the form of particles which, once hydrated, swell with the formation of soft beads.

The average particle size of the particles of the (d) superabsorbent polymer is 5 µm or more, preferably 10 µm or more, and more preferably 15 µm or more.

The average particle size of the particles of the (d) superabsorbent polymer is 500 µm or less, preferably 400 µm or less, and more preferably 300 µm or less.

Thus, the average particle size of the particles of the (d) superabsorbent polymer is 5 µm to 500 µm, preferably 10 µm to 400 µm, and more preferably 15 µm to 300 µm.

The average particle according to the present invention is a number-average particle size.

The number-average particle size is determined by a microscopy. The average particle size may be represented by a Heywood diameter.

If a particle of the (d) superabsorbent polymer is an aggregate of primary particles, the particle size here means the size of the aggregate. Thus, in this case, the particle size means the size of a secondary particle.

Preferably, the (d) superabsorbent polymer used in the present invention may be provided in the form of spherical particles.

The (d) superabsorbent polymer used in the present invention may preferably be crosslinked acrylic homo- or copolymers which are preferably neutralized and which are provided in the particulate form.

As the (d) superabsorbent polymer, mention may be made of:
Crosslinked (meth)acrylic acid or (meth)acrylate polymers, preferably crosslinked homopolymers or copolymers of (meth)acrylic acid and/or (meth)acrylate, and more preferably crosslinked sodium polyacrylates, such as, for example, those sold under the names Octacare X100, X110 and RM100 by Avecia, those sold under the names Flocare GB300 and Flosorb 500 by SNF, those sold under the names Luquasorb 1003, Luquasorb 1010, Luquasorb 1280 and Luquasorb 1100 by BASF, those sold under the names Water Lock G400 and G430 (INCI name: Acrylamide/Sodium Acrylate Copolymer) by Grain Processing, or Aqua Keep 10 SH NF provided by Sumitomo Seika, or Aqupec MG N40R (INCI name: Sodium Carbomer) provided by Sumitomo Seika, those (INCI name: Carbomer) sold under the names Aqupec HV-501E, Aqupec 505E, Aqupec 505ED, Aqupec 801EG, Aqupec 801EG-300, Aqupec 805EG, Aqupec 805EG-300 and Aqupec SW-705E provided by Sumitomo Seika, and the names Carbopol 934 Polymer, Carbopol 940 Polymer, Carbopol 941 Polymer, Carbopol 980 Polymer, and Carbopol 981 Polymer provided by Lubrizol;
Starches grafted with acrylic or acrylate polymer(s) (homopolymer or copolymer) and in particular by sodium polyacrylate, such as those sold under the names Sanfresh ST-100C, ST100MC and IM-300MC by Sanyo Chemical Industries, or Makimousse 25 and Makimousse 12 by Daito Kasei Kogyo (INCI name: Sodium Polyacrylate Starch);
Hydrolysed starches grafted by an acrylic polymer (homopolymer or copolymer), in particular the acryloacrylamide/sodium acrylate copolymer, such as those sold under the names Water Lock A-240, A-180, B-204, D-223, A-100, C-200 and D-223 by Grain Processing (INCI name: Starch/Acrylamide/Sodium Acrylate Copolymer);
Polymers based on starch, on gum and on cellulose derivatives, such as those comprising starch, guar gum and sodium carboxymethyl cellulose, sold under the name Lysorb 220 by Lysac; and
their blends.

The (d) superabsorbent polymer may be selected from crosslinked (meth)acrylic or (meth)acrylate polymers, starches grafted with acrylic or acrylate polymer(s), and mixtures thereof.

Preferably, the (d) superabsorbent polymer may be chosen from crosslinked sodium polyacrylates in the form of particles having a number-average size (or mean diameter) of less than or equal to 100 microns, more preferably in the form of spherical particles. These polymers preferably have a water-absorbing capacity of 10 to 100 g/g, preferably of 20 to 80 g/g and better still of 50 to 70 g/g.

The amount of the (d) superabsorbent polymer(s) in the composition according to the present invention may be 0.01% by weight or more, preferably 0.05% by weight or more, and more preferably 0.1% by weight or more, relative to the total weight of the composition.

The amount of the (d) superabsorbent polymer(s) in the composition according to the present invention may be 15% by weight or less, preferably 10% by weight or less, and more preferably 5% by weight or less, relative to the total weight of the composition.

The amount of the (d) superabsorbent polymer(s) in the composition according to the present invention may be from 0.01% to 15% by weight, preferably from 0.05% to 10% by weight, and more preferably from 0.1% to 5% by weight, relative to the total weight of the composition.

### [Water]

The composition according to the present invention includes (e) water.

The amount of the (e) water in the composition according to the present invention may be 50% by weight or more, preferably 60% by weight or more, and more preferably 70% by weight or more, relative to the total weight of the composition.

The amount of the (e) water in the composition according to the present invention may be 95% by weight or less, preferably 90% by weight or less, and more preferably 85% by weight or less, relative to the total weight of the composition.

The amount of the (e) water in the composition according to the present invention may be from 50% to 95% by weight, preferably from 60% to 90% by weight, and more preferably from 70% to 85% by weight, relative to the total weight of the composition.

### [pH]

The pH of the composition according to the present invention may be from 3 to 9, preferably from 3.3 to 8.5, and more preferably from 3.5 to 8.

At a pH of from 3 to 9, the polyion complex formed by the ingredients (a) to (c) can be very stable.

The pH of the composition according to the present invention may be adjusted by adding at least one alkaline agent and/or at least one acid, other than the (c) non-polymeric acid having two or more pKa values or salt(s) thereof. The pH of the composition according to the present invention may also be adjusted by adding at least one buffering agent.

### (Alkaline Agent)

The composition according to the present invention may comprise at least one alkaline agent. Two or more alkaline agents may be used in combination. Thus, a single type of alkaline agent or a combination of different types of alkaline agents may be used.

The alkaline agent may be an inorganic alkaline agent. It is preferable that the inorganic alkaline agent be selected from the group consisting of ammonia; alkaline metal hydroxides; alkaline earth metal hydroxides; alkaline metal phosphates and monohydrogenophosphates such as sodium phosphate or sodium monohydrogen phosphate.

As examples of the inorganic alkaline metal hydroxides, mention may be made of sodium hydroxide and potassium hydroxide. As examples of the alkaline earth metal hydroxides, mention may be made of calcium hydroxide and magnesium hydroxide. As an inorganic alkaline agent, sodium hydroxide is preferable.

The alkaline agent may be an organic alkaline agent. It is preferable that the organic alkaline agent be selected from the group consisting of monoamines and derivatives thereof; diamines and derivatives thereof; polyamines and derivatives thereof; basic amino acids and derivatives thereof; oligomers of basic amino acids and derivatives thereof; polymers of basic amino acids and derivatives thereof; urea and derivatives thereof; and guanidine and derivatives thereof.

As examples of the organic alkaline agents, mention may be made of alkanolamines such as mono-, di- and tri-ethanolamine, and isopropanolamine; urea, guanidine and their derivatives; basic amino acids such as lysine, ornithine or arginine; and diamines such as those described by the structure below: 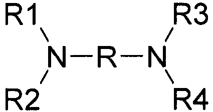 wherein R denotes an alkylene such as propylene, optionally substituted by a hydroxyl or a C₁-C₄ alkyl radical, and R₁, R₂, R₃ and R₄ independently denote a hydrogen atom, an alkyl radical or a C₁-C₄ hydroxyalkyl radical, which may be exemplified by 1,3-propanediamine and derivatives thereof. Arginine, urea and monoethanolamine are preferable.

The alkaline agent(s) may be used in a total amount of from 0.01% to 15% by weight, preferably from 0.02% to 10% by weight, more preferably from 0.03% to 5% by weight, relative to the total weight of the composition, depending on their solubility.

### (Acid)

The composition according to the present invention may comprise at least one acid. Two or more acids may be used in combination. Thus, a single type of acid or a combination of different types of acids may be used.

As the acid, mention may be made of any inorganic or organic acids, preferably inorganic acids, which are commonly used in cosmetic products. A monovalent acid and/or a polyvalent acid may be used. A monovalent acid such as citric acid, lactic acid, sulfuric acid, phosphoric acid and hydrochloric acid (HCl) may be used. HCl is preferable.

The acid(s) may be used in a total amount of from 0.01% to 15% by weight, preferably from 0.02% to 10% by weight, more preferably from 0.03% to 5% by weight, relative to the total weight of the composition, depending on their solubility.

### (Buffering Agent)

The composition according to the present invention may comprise at least one buffering agent. Two or more buffering agents may be used in combination. Thus, a single type of buffering agent or a combination of different types of buffering agents may be used.

As the buffering agent, mention may be made of an acetate buffer (for example, acetic acid + sodium acetate), a phosphate buffer (for example, sodium dihydrogen phosphate + di-sodium hydrogen phosphate), a citrate buffer (for example, citric acid + sodium citrate), a borate buffer (for example, boric acid + sodium borate), a tartrate buffer (for example, tartaric acid + sodium tartrate dihydrate), a Tris buffer (for example, tris(hydroxymethyl)aminomethane), and a Hepes buffer (4-(2-hydroxyethyl)-1-piperazineethanesulfonic acid).

### [Oil]

The composition according to the present invention may include (f) at least one oil. If two or more oils are used, they may be the same or different.

Here, "oil" means a fatty compound or substance which is in the form of a liquid or a paste (non-solid) at room temperature (25°C) under atmospheric pressure (760 mmHg). As the oils, those generally used in cosmetics can be used alone or in combination thereof. These oils may be volatile or non-volatile.

The (f) oil may be a non-polar oil such as a hydrocarbon oil, a silicone oil, or the like; a polar oil such as a plant or animal oil and an ester oil or an ether oil; or a mixture thereof.

The (f) oil may be selected from the group consisting of oils of plant or animal origin, synthetic oils, silicone oils, hydrocarbon oils and fatty alcohols.

As examples of plant oils, mention may be made of, for example, apricot oil, linseed oil, camellia oil, macadamia nut oil, corn oil, mink oil, olive oil, avocado oil, sasanqua oil, castor oil, safflower oil, jojoba oil, sunflower oil, almond oil, rapeseed oil, sesame oil, soybean oil, peanut oil, and mixtures thereof.

As examples of animal oils, mention may be made of, for example, squalene and squalane.

As examples of synthetic oils, mention may be made of alkane oils such as isododecane and isohexadecane, ester oils, ether oils, and artificial triglycerides.

The ester oils are preferably liquid esters of saturated or unsaturated, linear or branched C₁-C₂₆ aliphatic monoacids or polyacids and of saturated or unsaturated, linear or branched C₁-C₂₆ aliphatic monoalcohols or polyalcohols, the total number of carbon atoms of the esters being greater than or equal to 10.

Preferably, for the esters of monoalcohols, at least one is branched from among the alcohol and the acid from which the esters of the present invention are derived.

Among the monoesters of monoacids and of monoalcohols, mention may be made of ethyl palmitate, ethyl hexyl palmitate, isopropyl palmitate, dicaprylyl carbonate, alkyl myristates such as isopropyl myristate or ethyl myristate, isocetyl stearate, 2-ethylhexyl isononanoate, isononyl isononanoate, isodecyl neopentanoate and isostearyl neopentanoate.

Esters of C₄-C₂₂ dicarboxylic or tricarboxylic acids and of C₁-C₂₂ alcohols, and esters of monocarboxylic, dicarboxylic or tricarboxylic acids and of non-sugar C₄-C₂₆ dihydroxy, trihydroxy, tetrahydroxy or pentahydroxy alcohols may also be used.

Mention may especially be made of: diethyl sebacate; isopropyl lauroyl sarcosinate; diisopropyl sebacate; bis(2-ethylhexyl) sebacate; diisopropyl adipate; di-n-propyl adipate; dioctyl adipate; bis(2-ethylhexyl) adipate; diisostearyl adipate; bis(2-ethylhexyl) maleate; triisopropyl citrate; triisocetyl citrate; triisostearyl citrate; glyceryl trilactate; glyceryl trioctanoate; trioctyldodecyl citrate; trioleyl citrate; neopentyl glycol diheptanoate; diethylene glycol diisononanoate.

As ester oils, one can use sugar esters and diesters of C₆-C₃₀ and preferably C₁₂-C₂₂ fatty acids. It is recalled that the term "sugar" means oxygen-bearing hydrocarbon-based compounds containing several alcohol functions, with or without aldehyde or ketone functions, and which comprise at least 4 carbon atoms. These sugars may be monosaccharides, oligosaccharides or polysaccharides.

Examples of suitable sugars that may be mentioned include sucrose (or saccharose), glucose, galactose, ribose, fucose, maltose, fructose, mannose, arabinose, xylose and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may have one to three conjugated or non-conjugated carbon-carbon double bonds.

The esters according to this variant may also be selected from monoesters, diesters, triesters, tetraesters and polyesters, and mixtures thereof.

These esters may be, for example, oleates, laurates, palmitates, myristates, behenates, cocoates, stearates, linoleates, linolenates, caprates and arachidonates, or mixtures thereof such as, especially, oleopalmitate, oleostearate and palmitostearate mixed esters, as well as pentaerythrityl tetraethyl hexanoate.

More particularly, use is made of monoesters and diesters and especially sucrose, glucose or methylglucose monooleates or dioleates, stearates, behenates, oleopalmitates, linoleates, linolenates and oleostearates.

An example that may be mentioned is the product sold under the name Glucate^{®} DO by the company Amerchol, which is a methylglucose dioleate.

As examples of preferable ester oils, mention may be made of, for example, diisopropyl adipate, dioctyl adipate, 2-ethylhexyl hexanoate, ethyl laurate, cetyl octanoate, octyldodecyl octanoate, isodecyl neopentanoate, myristyl propionate, 2-ethylhexyl 2-ethylhexanoate, 2-ethylhexyl octanoate, 2-ethylhexyl caprylate/caprate, methyl palmitate, ethyl palmitate, isopropyl palmitate, dicaprylyl carbonate, isopropyl lauroyl sarcosinate, isononyl isononanoate, ethylhexyl palmitate, isohexyl laurate, hexyl laurate, isocetyl stearate, isopropyl isostearate, isopropyl myristate, isodecyl oleate, glyceryl tri(2-ethylhexanoate), pentaerythrityl tetra(2-ethylhexanoate), 2-ethylhexyl succinate, diethyl sebacate, cetearyl isononanoate, and mixtures thereof.

As examples of artificial triglycerides, mention may be made of, for example, capryl caprylyl glycerides, glyceryl trimyristate, glyceryl tripalmitate, glyceryl trilinolenate, glyceryl trilaurate, glyceryl tricaprate, glyceryl tricaprylate, glyceryl tri(caprate/caprylate) and glyceryl tri(caprate/caprylate/linolenate).

As examples of silicone oils, mention may be made of, for example, linear organopolysiloxanes such as dimethylpolysiloxane, methylphenylpolysiloxane, methylhydrogenpolysiloxane, and the like; cyclic organopolysiloxanes such as cyclohexasiloxane, octamethylcyclotetrasiloxane, decamethylcyclopentasiloxane, dodecamethylcyclohexasiloxane, and the like; and mixtures thereof.

Preferably, silicone oil is chosen from liquid polydialkylsiloxanes, especially liquid polydimethylsiloxanes (PDMS) and liquid polyorganosiloxanes comprising at least one aryl group.

These silicone oils may also be organomodified. The organomodified silicones that can be used for the present invention are silicone oils as defined above and comprise in their structure one or more organofunctional groups attached via a hydrocarbon-based group.

Organopolysiloxanes are defined in greater detail in Walter Noll's Chemistry and Technology of Silicones (1968), Academic Press. They may be volatile or non-volatile.

When they are volatile, the silicones are more particularly chosen from those having a boiling point of between 60°C and 260°C, and even more particularly from:
(i) Cyclic polydialkylsiloxanes comprising from 3 to 7 and preferably 4 to 5 silicon atoms. These are, for example, octamethylcyclotetrasiloxane sold in particular under the name Volatile Silicone^{®} 7207 by Union Carbide or Silbione^{®} 70045 V2 by Rhodia, decamethylcyclopentasiloxane sold under the name Volatile Silicone^{®} 7158 by Union Carbide, Silbione^{®} 70045 V5 by Rhodia, and dodecamethylcyclopentasiloxane sold under the name Silsoft 1217 by Momentive Performance Materials, and mixtures thereof. Mention may also be made of cyclocopolymers of the type such as dimethylsiloxane/methylalkylsiloxane, such as Silicone Volatile^{®} FZ 3109 sold by the company Union Carbide, of the formula:
   with 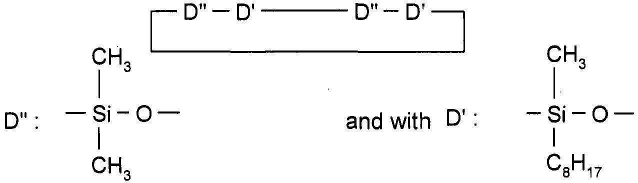
   Mention may also be made of mixtures of cyclic polydialkylsiloxanes with organosilicon compounds, such as the mixture of octamethylcyclotetrasiloxane and
   tetratrimethylsilylpentaerythritol (50/50) and the mixture of octamethylcyclotetrasiloxane and oxy-1,1'-bis(2,2,2',2',3,3'-hexatrimethylsilyloxy)neopentane; and
(ii) Linear volatile polydialkylsiloxanes containing 2 to 9 silicon atoms and having a viscosity of less than or equal to 5×10⁻⁶ m²/s at 25°C. An example is decamethyltetrasiloxane sold in particular under the name SH 200 by the company Toray Silicone. Silicones belonging to this category are also described in the article published in Cosmetics and Toiletries, Vol. 91, Jan. 76, pp. 27-32, Todd & Byers, Volatile Silicone Fluids for Cosmetics*.* The viscosity of the silicones is measured at 25°C according to ASTM standard 445 Appendix C.

Non-volatile polydialkylsiloxanes may also be used. These non-volatile silicones are more particularly chosen from polydialkylsiloxanes, among which mention may be made mainly of polydimethylsiloxanes containing trimethylsilyl end groups.

Among these polydialkylsiloxanes, mention may be made, in a non-limiting manner, of the following commercial products:
- the Silbione^{®} oils of the 47 and 70 047 series or the Mirasil^{®} oils sold by Rhodia, for instance the oil 70 047 V 500 000;
- the oils of the Mirasil^{®} series sold by the company Rhodia;
- the oils of the 200 series from the company Dow Corning, such as DC200 with a viscosity of 60,000 mm²/s; and
- the Viscasil^{®} oils from General Electric and certain oils of the SF series (SF 96, SF 18) from General Electric.

Mention may also be made of polydimethylsiloxanes containing dimethylsilanol end groups known under the name dimethiconol (CTFA), such as the oils of the 48 series from the company Rhodia.

Among the silicones containing aryl groups, mention may be made of polydiarylsiloxanes, especially polydiphenylsiloxanes and polyalkylarylsiloxanes such as phenyl silicone oil.

The phenyl silicone oil may be chosen from the phenyl silicones of the following formula: 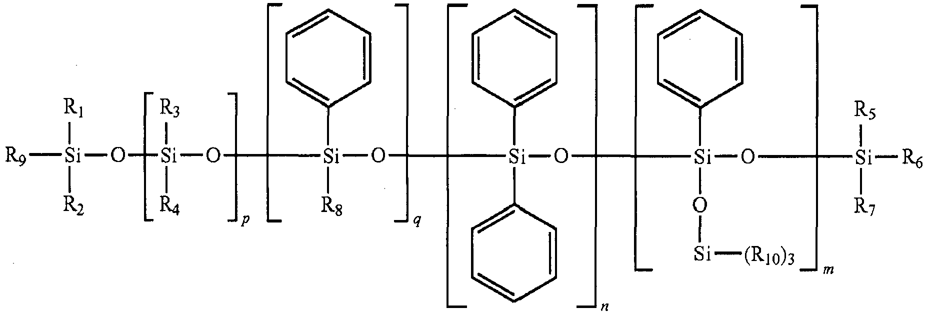 in which
R₁ to R₁₀, independently of each other, are saturated or unsaturated, linear, cyclic or branched C₁-C₃₀ hydrocarbon-based radicals, preferably C₁-C₁₂ hydrocarbon-based radicals, and more preferably C₁-C₆ hydrocarbon-based radicals, in particular methyl, ethyl, propyl or butyl radicals, and
m, n, p and q are, independently of each other, integers from 0 to 900 inclusive, preferably 0 to 500 inclusive, and more preferably 0 to 100 inclusive,
with the proviso that the sum n+m+q is not 0.

Examples that may be mentioned include the products sold under the following names:
- the Silbione^{®} oils of the 70 641 series from Rhodia;
- the oils of the Rhodorsil^{®} 70 633 and 763 series from Rhodia;
- the oil Dow Corning 556 Cosmetic Grade Fluid from Dow Corning;
- the silicones of the PK series from Bayer, such as the product PK20;
- certain oils of the SF series from General Electric, such as SF 1023, SF 1154, SF 1250 and SF 1265.

As the phenyl silicone oil, phenyl trimethicone (R₁ to R₁₀ are methyl; p, q, and n = 0; m=1 in the above formula) is preferable.

The organomodified liquid silicones may especially contain polyethyleneoxy and/or polypropyleneoxy groups. Mention may thus be made of the silicone KF-6017 proposed by Shin-Etsu, and the oils Silwet^{®} L722 and L77 from the company Union Carbide.

Hydrocarbon oils may be chosen from:
- linear or branched, optionally cyclic, C₆-C₁₆ lower alkanes. Examples that may be mentioned include hexane, undecane, dodecane, tridecane, and isoparaffins, for instance isohexadecane, isododecane and isodecane; and
- linear or branched hydrocarbons containing more than 16 carbon atoms, such as liquid paraffins, liquid petroleum jelly, polydecenes and hydrogenated polyisobutenes such as Parleam^{®}, and squalane.

As preferable examples of hydrocarbon oils, mention may be made of, for example, linear or branched hydrocarbons such as isohexadecane, isododecane, squalane, mineral oil (e.g., liquid paraffin), paraffin, vaseline or petrolatum, naphthalenes, and the like; hydrogenated polyisobutene, isoeicosan, and decene/butene copolymer; and mixtures thereof.

The term "fatty" in the fatty alcohol means the inclusion of a relatively large number of carbon atoms. Thus, alcohols which have 4 or more, preferably 6 or more, and more preferably 12 or more carbon atoms are encompassed within the scope of fatty alcohols. The fatty alcohol may be saturated or unsaturated. The fatty alcohol may be linear or branched.

The fatty alcohol may have the structure R-OH wherein R is chosen from saturated and unsaturated, linear and branched radicals containing from 4 to 40 carbon atoms, preferably from 6 to 30 carbon atoms, and more preferably from 12 to 20 carbon atoms. In at least one embodiment, R may be chosen from C₁₂-C₂₀ alkyl and C₁₂-C₂₀ alkenyl groups. R may or may not be substituted with at least one hydroxyl group.

As examples of the fatty alcohol, mention may be made of lauryl alcohol, cetyl alcohol, stearyl alcohol, isostearyl alcohol, behenyl alcohol, undecylenyl alcohol, myristyl alcohol, octyldodecanol, hexyldecanol, oleyl alcohol, linoleyl alcohol, palmitoleyl alcohol, arachidonyl alcohol, erucyl alcohol, and mixtures thereof.

It is preferable that the fatty alcohol be a saturated fatty alcohol.

Thus, the fatty alcohol may be selected from straight or branched, saturated or unsaturated C₆-C₃₀ alcohols, preferably straight or branched, saturated C₆-C₃₀ alcohols, and more preferably straight or branched, saturated C₁₂-C₂₀ alcohols.

The term "saturated fatty alcohol" here means an alcohol having a long aliphatic saturated carbon chain. It is preferable that the saturated fatty alcohol be selected from any linear or branched, saturated C₆-C₃₀ fatty alcohols. Among the linear or branched, saturated C₆-C₃₀ fatty alcohols, linear or branched, saturated C₁₂-C₂₀ fatty alcohols may preferably be used. Any linear or branched, saturated C₁₆-C₂₀ fatty alcohols may more preferably be used. Branched C₁₆-C₂₀ fatty alcohols may even more preferably be used.

As examples of saturated fatty alcohols, mention may be made of lauryl alcohol, cetyl alcohol, stearyl alcohol, isostearyl alcohol, behenyl alcohol, undecylenyl alcohol, myristyl alcohol, octyldodecanol, hexyldecanol, and mixtures thereof. In one embodiment, cetyl alcohol, stearyl alcohol, octyldodecanol, hexyldecanol, or a mixture thereof (e.g., cetearyl alcohol) as well as behenyl alcohol, can be used as a saturated fatty alcohol.

According to at least one embodiment, the fatty alcohol used in the composition according to the present invention is preferably chosen from octyldodecanol, hexyldecanol and mixtures thereof.

According to the present invention, the (f) oil may be surrounded by a plurality of the polyion complex particles or the (f) oil may be present in the hollow portion of a capsule formed by the polyion complex particles. In other words, the (f) oil may be covered by the polyion complex particles, or a capsule formed by the polyion complex particles includes the (f) oil in the hollow portion of the capsule.

The (f) oil which is surrounded by the polyion complex particles or present in the hollow portion of the capsule formed by the polyion complex particles cannot directly make contact with a keratin substance such as skin. Thus, even if the (f) oil has a sticky or greasy feeling of use, the composition according to the present invention will not provide a sticky or greasy feeling of use.

The amount of the (f) oil(s) in the composition according to the present invention may be 0.01% by weight or more, preferably 0.05% by weight or more, and more preferably 0.1% by weight or more, relative to the total weight of the composition.

The amount of the (f) oil(s) in the composition according to the present invention may be 50% by weight or less, preferably 40% by weight or less, and more preferably 30% by weight or less, relative to the total weight of the composition.

The amount of the (f) oil(s) in the composition according to the present invention may be from 0.01% to 50% by weight, preferably from 0.05% to 40% by weight, and more preferably from 0.1% to 30% by weight, relative to the total weight of the composition.

### [Optional Ingredients]

The composition according to the present invention may comprise, in addition to the aforementioned essential ingredients, at least one optional ingredient typically employed in cosmetics, which may be selected from, specifically, surfactants or emulsifiers, hydrophilic or lipophilic thickeners, organic volatile or non-volatile solvents such as polyols, in particular glycols, silicones and silicone derivatives other than the (f) oil, natural extracts derived from animals or vegetables, waxes, fillers, UV filters, and the like, within a range which does not impair the effects of the present invention.

The composition according to the present invention may comprise the above optional ingredient(s) in an amount of from 0.01% to 50% by weight, preferably from 0.05% to 30% by weight, and more preferably from 0.1% to 10% by weight, relative to the total weight of the composition.

However, it is preferable that the composition according to the present invention include a very limited amount of surfactant(s) or emulsifier(s). The amount of the surfactant(s) or emulsifier(s) in the composition according to the present invention may be 1% by weight or less, preferably 0.1% by weight or less, and more preferably 0.01% by weight or less, relative to the total weight of the composition. It is in particular preferable that the composition according to the present invention includes no surfactant or emulsifier.

### [Composition]

The composition according to the present invention may be intended to be used as a cosmetic composition. Thus, the cosmetic composition according to the present invention may be intended for application onto a keratin substance. Keratin substance here means a material containing keratin as a main constituent element, and examples thereof include the skin, scalp, nails, lips, hair, and the like. Thus, it is preferable that the cosmetic composition according to the present invention be used for a cosmetic process for the keratin substance, in particular skin.

Thus, the cosmetic composition according to the present invention may be a skin cosmetic composition, preferably a skin care composition or a skin makeup composition, and more preferably a skin care composition.

The composition according to the present invention can be prepared by mixing the above essential and optional ingredients in accordance with any of the processes which are well known to those skilled in the art.

The composition according to the present invention can be prepared by simple or easy mixing with a conventional mixing means such as a stirrer. Thus, strong shearing by, for example, a homogenizer is not necessary. Also, heating is not necessary.

If the composition according to the present invention includes the (f) oil(s), it can be in the form of an emulsion, an O/W emulsion or a W/O emulsion. It is preferable that the composition according to the present invention be in the form of an O/W emulsion, because it can provide a fresh sensation due to the (e) water which forms the outer phase thereof.

It is more preferable that the amount of the surfactant(s) or emulsifier(s) in the emulsion, in particular an O/W emulsion, be 3% by weight or less, preferably 2% by weight or less, and more preferably 1% by weight or less, relative to the total weight of the composition, because the surfactant(s) may negatively affect water-resistance. It is in particular preferable that the emulsion, in particular an O/W emulsion includes no surfactant or emulsifier.

### [Film]

The composition according to the present invention can be used for easily preparing a film. This film is not part of the invention as claimed. The (a) cationic polymer and the (b) anionic polymer, as well as the (c) non-polymeric acid having two or more pKa values or salt(s) thereof, which can form a polyion complex, can integrate to form a continuous film.

Thus, a process for preparing a film, preferably a cosmetic film, optionally with a thickness of preferably more than 0.1 µm, more preferably 1.5 µm or more, and even more preferably 2 µm or more, comprising:
applying onto a substrate, preferably a keratin substance, more preferably skin, the composition according to the present invention; and
drying the composition, is also disclosed.

The upper limit of the thickness of the film obtained with the present invention is not limited.

Thus, for example, the thickness of the film may be 1 mm or less, preferably 500 µm or less, more preferably 300 µm or less, and even more preferably 100 µm or less.

Since the above process for preparing the film includes the steps of applying the composition according to the present invention onto a substrate, preferably a keratin substance, and more preferably skin, and of drying the composition, the process according to the present invention does not require any spin coating or spraying, and therefore, it is possible to easily prepare even a relatively thick film. Thus, the process for preparing the film can prepare a relatively thick film without any special equipment such as spin coaters and spraying machines.

Even if the film is relatively thick, it is still thin and may be transparent, and therefore, may not be easy to perceive.

If the substrate is not a keratin substance such as skin, the composition according to the present invention may be applied onto a substrate made from any material other than keratin. The materials of the non-keratinous substrate are not limited. Two or more materials may be used in combination. Thus, a single type of material or a combination of different types of materials may be used. In any event, it is preferable that the substrate be flexible or elastic.

If the substrate is not a keratin substance, it is preferable that the substrate be water-soluble, because it is possible to leave the film by washing the substrate with water. As examples of the water-soluble materials, mention may be made of poly(meth) acrylic acids, polyethyleneglycols, polyacrylamides, polyvinylalcohol (PVA), starch, celluloseacetates, and the like. PVA is preferable.

If the non-keratinous substrate is in the form of a sheet, it may have a thickness of more than that of the film, in order to ease the handling of the film attached to the substrate sheet. The thickness of the non-keratinous substrate sheet is not limited, but may be from 1 µm to 5 mm, preferably from 10 µm to 1 mm, and more preferably from 50 to 500 µm.

It is more preferable that the film according to the present invention be releasable from the non-keratinous substrate. The mode of release is not limited. Therefore, the film may be peeled from the non-keratinous substrate, or released by the dissolution of the substrate sheet into a solvent such as water.

Processes wherein the compositions of the invention are applied on non-keratinic substance are not part of the invention as claimed.

The composition of the invention may lead to the following films :
(1) A film, preferably a cosmetic film, optionally with a thickness of preferably more than 0.1 µm, more preferably 1.5 µm or more, and even more preferably 2 µm or more, prepared by a process comprising:
   applying onto a substrate, preferably a keratin substance, and more preferably skin, the composition according to the present invention; and
   drying the composition,
   and
(2) A film, preferably a cosmetic film, optionally with a thickness of preferably more than 0.1 µm, more preferably 1.5 µm or more, and even more preferably 2 µm or more, comprising:
   (a) at least one cationic polymer;
   (b) at least one anionic polymer;
   (c) at least one non-polymeric acid having two or more pKa values or salt(s) thereof; and

   optionally (f) at least one oil,
   wherein
   the (b) anionic polymer is selected from hyaluronic acids and salts thereof.

The above explanations regarding the (a) cationic and the (b) anionic polymers as well as the (c) non-polymeric acid having two or more pKa values or salt(s) thereof and the (f) oil can apply to those in the above films (1) and (2).

The film thus obtained above can be self-standing. The term "self-standing" here means that the film can be in the form of a sheet and can be handled as an independent sheet without the assistance of a substrate or support. Thus, the term "self-standing" may have the same meaning as "self-supporting".

It is preferable that the above film be hydrophobic.

The term "hydrophobic" in the present specification means that the solubility of the polymer in water (preferably with a volume of 1 liter) at from 20 to 40°C, preferably from 25 to 40°C, and more preferably from 30 to 40°C is less than 10% by weight, preferably less than 5% by weight, more preferably less than 1% by weight, and even more preferably less than 0.1% by weight, relative to the total weight of the polymer. It is most preferable that the polymer is not soluble in water.

If the above film is hydrophobic, the film can have water-resistant properties, and therefore, it can remain on a keratin substance such as skin, even if the surface of the keratin substance is wet due to, for example, sweat or rain. Thus, when the film provides any cosmetic effect, the cosmetic effect can last a long time.

On the other hand, the film can be easily removed from a keratin substance such as skin under alkaline conditions such as a pH of from 8 to 12, preferably from 9 to 11. Therefore, the film is difficult to remove with water, while it can be easily removed with a soap which can provide such alkaline conditions.

The film may comprise at least one biocompatible and/or biodegradable polymer layer. Two or more biocompatible and/or biodegradable polymers may be used in combination. Thus, a single type of biocompatible and/or biodegradable polymer or a combination of different types of biocompatible and/or biodegradable polymers may be used.

The term "biocompatible" polymer in the present specification means that the polymer does not have excess interaction between the polymer and cells in the living body including the skin, and the polymer is not recognized by the living body as a foreign material.

The term "biodegradable" polymer in the present specification means that the polymer can be degraded or decomposed in a living body due to, for example, the metabolism of the living body itself or the metabolism of the microorganisms which may be present in the living body. Also, the biodegradable polymer can be degraded by hydrolysis.

If the film includes a biocompatible and/or biodegradable polymer, it is less irritable or not irritable to the skin, and does not cause any rash. In addition, due to the use of a biocompatible and/or biodegradable polymer, the film can adhere well to the skin.

The film can be used for cosmetic treatments of keratin substances, preferably skin, in particular the face. The film can be in any shape or form. For example, it can be used as a full-face mask sheet, or a patch for a part of the face such as the cheek, nose, and around the eyes.

If the film includes at least one hydrophilic or water-soluble UV filter, it can provide UV shielding effects derived from the hydrophilic or water-soluble UV filter. Normally, a hydrophilic or water-soluble UV filter can be removed from the surface of a keratinous substrate such as skin by water such as sweat and rain. However, since the hydrophilic or water-soluble UV filter is included in the film, it is difficult for the hydrophilic or water-soluble UV filter to be removed by water, thereby resulting in long-lasting UV shielding effects.

### [Cosmetic Process]

The present invention also relates to:
a cosmetic process for a keratin substance such as skin, comprising: applying to the keratin substance the composition according to the present invention; and drying the composition to form a cosmetic film on the keratin substance.

The cosmetic process here means a non-therapeutic cosmetic method for caring for and/or making up the surface of a keratin substance such as skin.

In the above process, the above cosmetic film is resistant to water with a pH of 7 or less, and is removable with water with a pH of more than 7, preferably 8 or more, and more preferably 9 or more.

In other words, the above cosmetic film can be water-resistant under neutral or acidic conditions such as a pH of 7 or less, preferably in a range of 6 or more and 7 or less, and more preferably in a range of 5 or more and 7 or less, while the above cosmetic film can be removed under alkaline conditions such as a pH of more than 7, preferably 8 or more, and more preferably 9 or more. The upper limit of the pH is preferably 13, more preferably 12, and even more preferably 11.

Accordingly, the above cosmetic film can be water-resistant, and therefore, it can remain on a keratin substance such as skin even if the surface of the keratin substance is wet due to, for example, sweat or rain. On the other hand, the above cosmetic film can be easily removed from a keratin substance such as skin under alkaline conditions. Therefore, the film is difficult to remove with water, while it can be easily removed with a soap which can provide alkaline conditions.

If the above cosmetic film includes a UV filter which may be present in the composition according to the present invention, the above cosmetic film can protect a keratin substance such as skin from UV rays, thereby limiting the darkening of the skin, improving the colour and uniformity of the complexion, and/or treating aging of the skin.

Furthermore, the above cosmetic film may have cosmetic effects such as capturing sebum, matting the appearance of a keratin substrate such as skin, absorbing or adsorbing malodour, and/or protecting the keratin substance from, for example, dirt or pollutant, due to the properties of the polyion complex in the cosmetic film, even if the cosmetic film does not include any cosmetic active ingredient.

In addition, the above cosmetic film may immediately change or modify the appearance of the skin by changing light reflection on the skin and the like, even if the cosmetic film does not include any cosmetic active ingredient. Therefore, it may be possible for the above cosmetic film to conceal skin defects such as pores or wrinkles. Further, the above cosmetic film may immediately change or modify the feel to the touch of the skin by changing the surface roughness on the skin and the like. Furthermore, the above cosmetic film may immediately protect the skin by covering the surface of the skin and shielding the skin, as a barrier, from environmental stresses such as pollutants, contaminants and the like.

The above cosmetic effects can be adjusted or controlled by changing the chemical composition, the thickness and/or the surface roughness of the above cosmetic film.

If the above cosmetic film includes at least one additional cosmetic active ingredient other than the (f) oil, the cosmetic film can have cosmetic effects provided by the additional cosmetic active ingredient(s). For example, if the cosmetic film includes at least one cosmetic active ingredient selected from anti-aging agents, anti-sebum agents, deodorant agents, antiperspirant agents, whitening agents and a mixture thereof, the cosmetic film can treat the aging of the skin, absorbing sebum on the skin, controlling odors on the skin, controlling perspiration on the skin, and/or whitening of the skin.

It is also possible to apply a makeup cosmetic composition onto the cosmetic film or sheet after it has been applied onto the skin.

### EXAMPLES

The present invention will be described in a more detailed manner by way of examples.

### [Examples 1-3 and Comparative Examples 1-4]

### [Preparation]

Each of the compositions according to Examples 1-3 and Comparative Examples 1-4 was prepared by mixing the ingredients shown in Table 1 in accordance with the following Steps 1-8.
1. The ingredients for Phase A were mixed and homogenized at 75°C+/-5°C to obtain a mixture of Phase A.
2. The ingredients for Phase B were added to the mixture of Phase A obtained by Step 1 and homogenized at 75°C+/-5°C to obtain a mixture of Phases A and B.
3. The ingredient for Phase C was added to the above mixture of Phases A and B obtained by Step 2 and homogenized at 75°C+/-5°C to obtain a mixture of Phases A, B and C*.*
4. The ingredient for Phase D was added to the above mixture of Phases A, B and C obtained by Step 3 and homogenized at 75°C+/-5°C to obtain a mixture of Phases A, B, C and D.
5. The ingredient for Phase E was added to the above mixture of Phases A, B, C and D obtained by Step 4 and homogenized at 75°C+/-5°C to obtain a mixture of Phases A, B, C, D and E.
6. The ingredients for Phase F were added to the mixture of Phases A, B, C, D and E obtained by Step 5, and homogenized at 75°C+/-5°C to obtain a mixture of Phases A, B. C. D. E and F.
7. The ingredient for Phase G was added to the above mixture of Phases A, B, C, D, E and F obtained by Step 6, and homogenized at room temperature to obtain a mixture of Phases A, B, C, D, E, F and G.
8. The ingredient for Phase H was added to the above mixture of Phases A, B, C, D, E, F and G obtained by Step 7 and homogenized at room temperature to obtain a homogeneous composition.

The numerical values for the amounts of the ingredients in Table 1 are all based on "% by weight" as active materials.

The (a) to (f) in Table 1 correspond to those in the claims.

**Table 1**

| Phase | | | Ex. 1 | Ex.2 | Ex.3 | Comp. Ex. 1 | Comp. Ex. 2 | Comp. Ex. 3 | Comp. Ex. 4 |
|---|---|---|---|---|---|---|---|---|---|
| A | (e) | Water | qsp 100 | qsp 100 | qsp 100 | qsp 100 | qsp 100 | qsp 100 | qsp 100 |
| | | Propanediol | 3.00 | 3.00 | 3.00 | 3.00 | 3.00 | 3.00 | 3.00 |
| | | Pentylene Glycol | 3.00 | 3.00 | 3.00 | 3.00 | 3.00 | 3.00 | 3.00 |
| | | Chlorphenesin | 0.27 | 0.27 | 0.27 | 0.27 | 0.27 | 0.2 7 | 0.27 |
| | | Trisodium Ethylenediamine Disuccinate | 0.20 | 0.20 | 020 | 0.20 | 0.20 | 0.20 | 0.20 |
| | | Betaine | 3.00 | 3.00 | 3.00 | 3.00 | 3.00 | 3.00 | 3.00 |
| B | | Glyceryl Stearate Citrate | 0.50 | 0.50 | 0.50 | 0.50 | 0.50 | 0.50 | 0.50 |
| | | Polyglyceryl-4 Caprate | 0.20 | 0.20 | 0.20 | 0.20 | 0.20 | 0.20 | 0.20 |
| | (f) | Behenyl Alcohol | 2.00 | 2.00 | 2.00 | 2.00 | 2.00 | 2.00 | 2.00 |
| | (f) | Isopropyl Myristate | 5.00 | 5.00 | 5.00 | 5.00 | 5.00 | 5.00 | 5.00 |
| | (f) | Cetearyl Isononanoate | 0.50 | 0.50 | 0.50 | 0.50 | 0.50 | 0.50 | 0.50 |
| | (f) | Squalane | 0.50 | 0.50 | 0.50 | 0.50 | 0.50 | 0.50 | 0.50 |
| C | (a) | Sodium Hyaluronate (1100 kDa) | 0.15 | 0.15 | 0.15 | - | 0.15 | 0.15 | 0.15 |
| | (a) | Sodium Hyaluronate (50 kDa) | 0.15 | 0.15 | 0.15 | - | 0.15 | 0.15 | 0.15 |
| D | (b) | Polyepsilon-Lysin | 0.60 | 0.60 | 0.60 | 0.60 | - | 0.60 | 0.60 |
| E | (c) | Phytic Acid | 0.17 | 0.17 | 0.17 | 0.17 | 0.17 | - | 0.17 |
| F | (d) | Sodium Polyacrylate Starch (particle size: 35 µm) | 0.75 | - | - | 0.75 | 0.75 | 0.75 | - |
| | (d) | Sodium Polyacrylate Starch (particle size: 183 µm) | - | 0.75 | - | - | - | - | - |
| | (d) | Sodium Carbomer (particle size: 90 µm) | - | - | 0.75 | - | - | - | - |
| | | Hectorite | 0.10 | 0.10 | 0.10 | 0.10 | 0.10 | 0.10 | 0.10 |
| | | Acrylates/C10-30 Alkyl Acrylate Crosspolymer | 0.10 | 0.10 | 0.10 | 0.10 | 0.10 | 0.10 | 0.10 |
| | | Xanthan Gum | 0.10 | 0.10 | 0.10 | 0.10 | 0.10 | 0.10 | 0.10 |
| G | | Silica Silylate | 0.30 | 0.30 | 0.30 | 0.30 | 0.30 | 0.30 | 0.30 |
| H | | Potassium Hydroxide | qs | qs | qs | qs | qs | qs | qs |
| Sebum Control | | | Good | Good | Good | Poor | Poor | Poor | Poor |
| Noodle | | | Good | Good | Good | Poor | Poor | Poor | Good |
| Stability | | | Good | Good | Good | Good | Good | Good | Poor |

| | | | | | | | | | |
|---|---|---|---|---|---|---|---|---|---|
| Particle size: number-average particle size determined by a microscopy | | | | | | | | | |

### [Evaluations]

### (Sebum Control)

Each of the compositions according to Examples 1-3 and Comparative Examples 1-4 was spread over a contrast sheet by an automatic film applicator to form a layer of 100 µm and left to dry at 37°C for 24 hours. Thereafter, an artificial sebum/sweat composition with the formulation shown in the following Table 2 was sprayed on the above layer on the contrast card, at room temperature. The amount of the artificial sebum/sweat composition sprayed on each of the above layers was the same as each other.

**Table 2**

| | (wt%) |
|---|---|
| Oleic acid | 20.0 |
| Poly(oxy-1,2-ethanediyl) | 1.0 |
| Water | 79.0 |
| Total | 100.0 |

After 24 hours, the surface roughness of the above layer was observed by a confocal microscope. The surface roughness was evaluated by the following criteria:
Good: High surface roughness
Poor: Low surface roughness

A high surface roughness means that the layer absorbed the artificial sebum/sweat composition and maintained it therein for a long period of time. The high surface roughness is also good to show a matte effect. Thus, the high surface roughness reflects good sebum control effects.

The compositions according to Examples 1-3 were able to form a sufficiently rough surface. On the other hand, the compositions according to Comparative Examples 1-4 could not form a sufficiently rough surface. These results reflect that a combination of a polyion complex and a superabsorbent polymer can provide enhanced anti-sebum effects which can contribute to a matte appearance.

### (Noodle)

The artificial sebum/sweat composition with the formulation shown in the above Table 2 was applied onto a bioskin. Next, each of the compositions according to Examples 1-3 and Comparative Examples 1-4 was applied onto the bioskin, and spread by a finger, followed by being dried at 40°C. Next, a foundation was applied thereon and rubbed. The formation of noodles of the foundation was evaluated by the following criteria.
Good: No or little noodles were observed
Poor: Noodles were remarkably observed
Noodles: Aggregation which appears as eraser residue produced during application

The compositions according to Examples 1-3 caused no or little noodles. On the other hand, the compositions according to Comparative Examples 1-3 caused a remarkable amount of noodles. These results reflect that the polyion complex can suppress the formation of noodles.

### (Stability)

Each of the compositions according to Examples 1-3 and Comparative Examples 1-4 was stored in a transparent vessel at 45°C for 2 months. The stability of the compositions was visually observed and evaluated in accordance with the following criteria.
Good: The uniformity of the composition was maintained.
Poor: The uniformity of the composition was not maintained (water release or phase separation was remarkably noticed).

The results are shown in Table 1.

The compositions according to Examples 1-3 were stable. On the other hand, the composition according to Comparative Example 4 was unstable. These results reflect that a superabsorbent polymer can enhance the stability of a composition including a polyion complex.

## Claims

1. A composition, comprising:
(a) at least one cationic polymer;
(b) at least one anionic polymer;
(c) at least one non-polymeric acid having two or more pKa values or salt(s) thereof;
(d) at least one superabsorbent polymer which is capable, in its dry state, of spontaneously absorbing at least 20 times its own weight of water ; and
(e) water,
wherein the (b) anionic polymer is selected from hyaluronic acids and salts thereof, and
the (d) superabsorbent polymer is in the form of particles with a number average particle size of 5 µm to 500 µm, preferably 10 µm to 400 µm, and more preferably 15 µm to 300 µm, the number average particle size being determined by a microscopy.

2. The composition according to Claim 1, wherein the (a) cationic polymer has at least one positively chargeable and/or positively charged moiety selected from the group consisting of a primary, secondary or tertiary amino group, a quaternary ammonium group, a guanidine group, a biguanide group, an imidazole group, an imino group, and a pyridyl group.

3. The composition according to Claim 1 or 2, wherein the (a) cationic polymer is selected from the group consisting of cyclopolymers of alkyldiallylamine and cyclopolymers of dialkyldiallylammonium such as (co)polydiallyldialkyl ammonium chloride, (co)polyamines such as chitosans and (co)polylysines, cationic (co)polyaminoacids such as collagen, cationic cellulose polymers, and salts thereof.

4. The composition according to any one of Claims 1 to 3, wherein the amount of the (a) cationic polymer(s) in the composition is from 0.01% to 15% by weight, preferably from 0.05% to 10% by weight, and more preferably from 0.1% to 5% by weight, relative to the total weight of the composition.

5. The composition according to any one of Claims 1 to 4, wherein the composition comprises at least two hyaluronic acids with different molecular weights, preferably a first hyaluronic acid with a molecular weight of 100 kDa or less and a second hyaluronic acid with a molecular weight of 500 kDa or more, and more preferably a first hyaluronic acid with a molecular weight of 50 kDa or less and a second hyaluronic acid with a molecular weight of 1000 kDa or more, as the (b) anionic polymers.

6. The composition according to any one of Claims 1 to 5, wherein the amount of the (b) anionic polymer(s) in the composition is from 0.01% to 15% by weight, preferably from 0.05% to 10% by weight, and more preferably from 0.1% to 5% by weight, relative to the total weight of the composition.

7. The composition according to any one of Claims 1 to 6, wherein the (c) non-polymeric acid having two or more pKa values or salt(s) thereof is an organic acid or salt(s) thereof, preferably a hydrophilic or water-soluble organic acid or salt(s) thereof, and more preferably phytic acid or salts thereof.

8. The composition according to any one of Claims 1 to 7, wherein the amount of the (c) non-polymeric acid having two or more pKa values or salt(s) thereof in the composition is from 0.01% to 15% by weight, preferably from 0.05% to 10% by weight, and more preferably from 0.1% to 5% by weight, relative to the total weight of the composition.

9. The composition according to any one of Claims 1 to 8, wherein the (d) superabsorbent polymer is selected from crosslinked (meth)acrylic or (meth)acrylate polymers, starches grafted with acrylic or acrylate polymer(s), and mixtures thereof.

10. The composition according to any one of Claims 1 to 9, wherein the amount of the (d) superabsorbent polymer in the composition is from 0.01% to 15% by weight, preferably from 0.05% to 10% by weight, and more preferably from 0.1% to 5% by weight, relative to the total weight of the composition.

11. The composition according to any one of Claims 1 to 10, wherein the amount of the (e) water in the composition is from 50% to 95% by weight, preferably from 60% to 90% by weight, and more preferably from 70% to 85% by weight, relative to the total weight of the composition.

12. The composition according to any one of Claims 1 to 11, wherein the pH of the composition is from 3 to 9, preferably from 3.3 to 8.0, and more preferably from 3.5to 8.

13. The composition according to any one of Claims 1 to 12, wherein the composition further comprises (f) at least one oil.

14. The composition according to any one of Claims 1 to 13, wherein the composition is a cosmetic composition, preferably a skin cosmetic composition, and more preferably a skin care cosmetic composition.

15. A cosmetic process for a keratin substance such as skin, comprising
applying to the keratin substance the composition according to any one of Claims 1 to 14; and
drying the composition to form a cosmetic film on the keratin substance.

## Patentansprüche

1. Zusammensetzung umfassend:
(a) wenigstens ein kationisches Polymer;
(b) wenigstens ein anionisches Polymer;
(c) wenigstens eine nichtpolymere Säure mit zwei oder mehr pKa-Werten oder Salz(e) davon;
(d) wenigstens ein superabsorbierendes Polymer, das fähig ist, in seinem trockenen Zustand wenigstens das 20-fache seines Eigengewichts an Wasser spontan zu absorbieren; und
(e) Wasser,
wobei das (b) anionische Polymer ausgewählt ist aus Hyaluronsäuren und Salzen davon, und
das (d) superabsorbierende Polymer in der Form von Partikeln mit einer zahlenmittleren Partikelgröße von 5 µm bis 500 µm, vorzugsweise 10 µm bis 400 µm und bevorzugter 15 µm bis 300 µm vorliegt, wobei die zahlenmittlere Partikelgröße durch ein Mikroskop bestimmt wird.

2. Zusammensetzung nach Anspruch 1, wobei das (a) kationische Polymer wenigstens eine positiv ladbare und/oder positiv geladene Einheit ausgewählt aus der Gruppe bestehend aus einer primären, sekundären oder tertiären Aminogruppe, einer quartären Ammoniumgruppe, einer Guanidingruppe, einer Biguanidgruppe, einer Imidazolgruppe, einer Iminogruppe und einer Pyridylgruppe aufweist.

3. Zusammensetzung nach Anspruch 1 oder 2, wobei das (a) kationische Polymer ausgewählt ist aus der Gruppe bestehend aus Cyclopolymeren von Alkyldiallylamin und Cyclopolymeren von Dialkyldiallylammonium, wie z. B. (Co)Polydiallyldialkylammoniumchlorid, (Co)Polyaminen, wie z. B. Chitosanen und (Co)Polylysinen, kationischen (Co)Polyaminosäuren, wie z. B. Collagen, kationischen Cellulosepolymeren und Salzen davon.

4. Zusammensetzung nach einem der Ansprüche 1 bis 3, wobei die Menge an den (a) kationischen Polymer(en) in der Zusammensetzung von 0,01 Gew.-% bis 15 Gew.-%, vorzugsweise von 0,05 Gew.-% bis 10 Gew.-% und bevorzugter von 0,1 Gew.-% bis 5 Gew.-%, bezogen auf das Gesamtgewicht der Zusammensetzung, beträgt.

5. Zusammensetzung nach einem der Ansprüche 1 bis 4, wobei die Zusammensetzung wenigstens zwei Hyaluronsäuren mit unterschiedlichen Molekulargewichten, vorzugsweise eine erste Hyaluronsäure mit einem Molekulargewicht von 100 kDa oder weniger und eine zweite Hyaluronsäure mit einem Molekulargewicht von 500 kDa oder mehr, und bevorzugter eine erste Hyaluronsäure mit einem Molekulargewicht von 50 kDa oder weniger und eine zweite Hyaluronsäure mit einem Molekulargewicht von 1000 kDa oder mehr, als die (b) anionischen Polymere umfasst.

6. Zusammensetzung nach einem der Ansprüche 1 bis 5, wobei die Menge an den (b) anionischen Polymer(en) in der Zusammensetzung von 0,01 Gew.-% bis 15 Gew.-%, vorzugsweise von 0,05 Gew.-% bis 10 Gew.-% und bevorzugter von 0,1 Gew.-% bis 5 Gew.-%, bezogen auf das Gesamtgewicht der Zusammensetzung, beträgt.

7. Zusammensetzung nach einem der Ansprüche 1 bis 6, wobei die (c) nichtpolymere Säure mit zwei oder mehr pKa-Werten oder Salz(e) davon eine organische Säure oder Salz(e) davon ist, vorzugsweise eine hydrophile oder wasserlösliche organische Säure oder Salz(e) davon und bevorzugter Phytinsäure oder Salze davon.

8. Zusammensetzung nach einem der Ansprüche 1 bis 7, wobei die Menge der (c) nichtpolymeren Säure mit zwei oder mehr pKa-Werten oder Salz(en) davon in der Zusammensetzung von 0,01 Gew.-% bis 15 Gew.-%, vorzugsweise von 0,05 Gew.-% bis 10 Gew.-% und bevorzugter von 0,1 Gew.-% bis 5 Gew.-%, bezogen auf das Gesamtgewicht der Zusammensetzung, beträgt.

9. Zusammensetzung nach einem der Ansprüche 1 bis 8, wobei das (d) superabsorbierende Polymer ausgewählt ist aus vernetzten (Meth)acryl- oder (Meth)acrylatpolymeren, mit Acryl- oder Acrylatpolymer(en) gepfropften Stärken und Gemischen davon.

10. Zusammensetzung nach einem der Ansprüche 1 bis 9, wobei die Menge an dem (d) superabsorbierenden Polymer in der Zusammensetzung von 0,01 Gew.-% bis 15 Gew.-%, vorzugsweise von 0,05 Gew.-% bis 10 Gew.-% und bevorzugter von 0,1 Gew.-% bis 5 Gew.-%, bezogen auf das Gesamtgewicht der Zusammensetzung, beträgt.

11. Zusammensetzung nach einem der Ansprüche 1 bis 10, wobei die Menge an dem (e) Wasser in der Zusammensetzung von 50 Gew.-% bis 95 Gew.-%, vorzugsweise von 60 Gew.-% bis 90 Gew.-% und bevorzugter von 70 Gew.-% bis 85 Gew.-%, bezogen auf das Gesamtgewicht der Zusammensetzung, beträgt.

12. Zusammensetzung nach einem der Ansprüche 1 bis 11, wobei der pH-Wert der Zusammensetzung von 3 bis 9, vorzugsweise 3,3 bis 8,0 und bevorzugter 3,5 bis 8 beträgt.

13. Zusammensetzung nach einem der Ansprüche 1 bis 12, wobei die Zusammensetzung ferner (f) wenigstens ein Öl umfasst.

14. Zusammensetzung nach einem der Ansprüche 1 bis 13, wobei die Zusammensetzung eine kosmetische Zusammensetzung, vorzugsweise eine hautkosmetische Zusammensetzung und bevorzugter eine kosmetische Hautpflegezusammensetzung ist.

15. Kosmetisches Verfahren für einen Keratinstoff, wie z. B. Haut, umfassend
Aufbringen der Zusammensetzung nach einem der Ansprüche 1 bis 14 auf den Keratinstoff; und
Trocknen der Zusammensetzung, um einen kosmetischen Film auf dem Keratinstoff zu bilden.

## Revendications

1. Composition, comprenant :
(a) au moins un polymère cationique ;
(b) au moins un polymère anionique ;
(c) au moins un acide non polymérique ayant au moins deux valeurs de pKa ou plus ou un ou plusieurs sels de celui-ci ;
(d) au moins un polymère superabsorbant qui est capable, dans son état sec, d'absorber spontanément au moins 20 fois son propre poids d'eau ; et
(e) de l'eau,
dans laquelle le polymère anionique (b) est choisi parmi les acides hyaluroniques et leurs sels, et
le polymère superabsorbant (d) se présente sous la forme de particules ayant une taille moyenne de particule en nombre de 5 µm à 500 µm, de préférence de 10 µm à 400 µm, et plus préférablement de 15 µm à 300 µm, la taille moyenne de particule en nombre étant déterminée par une microscopie.

2. Composition selon la revendication 1, dans laquelle le polymère cationique (a) possède au moins un groupement pouvant être chargé positivement et/ou chargé positivement choisi dans le groupe constitué par un groupe amino primaire, secondaire ou tertiaire, un groupe ammonium quaternaire, un groupe guanidine, un groupe biguanide, un groupe imidazole, un groupe imino, et un groupe pyridinyle.

3. Composition selon la revendication 1 ou 2, dans laquelle le polymère cationique (a) est choisi dans le groupe constitué par les cyclopolymères d'alkyldiallylamine et les cyclopolymères de dialkyldiallylammonium tels que le chlorure de (co)polydiallyldialkylammonium, les (co)polyamines telles que les chitosanes et les (co)polylysines, les (co)polyaminoacides cationiques tels que le collagène, les polymères de cellulose cationique et leurs sels.

4. Composition selon l'une quelconque des revendications 1 à 3, dans laquelle la quantité du ou des polymères cationiques (a) présents dans la composition est de 0,01 % à 15 % en poids, préférablement de 0,05 % à 10 % en poids, et plus préférablement de 0,1 % à 5 % en poids, par rapport au poids total de la composition.

5. Composition selon l'une quelconque des revendications 1 à 4, la composition comprenant au moins deux acides hyaluroniques ayant des poids moléculaires différents, de préférence un premier acide hyaluronique ayant un poids moléculaire de 100 kDa ou moins et un second acide hyaluronique ayant un poids moléculaire de 500 kDa ou plus, et plus préférablement, un premier acide hyaluronique ayant un poids moléculaire de 50 kDa ou moins et un second acide hyaluronique ayant un poids moléculaire de 1 000 kDa ou plus, en tant que polymères anioniques (b).

6. Composition selon l'une quelconque des revendications 1 à 5, dans laquelle la quantité du ou des polymères anioniques (b) présents dans la composition est de 0,01 % à 15 % en poids, préférablement de 0,05 % à 10 % en poids, et plus préférablement de 0,1 % à 5 % en poids, par rapport au poids total de la composition.

7. Composition selon l'une quelconque des revendications 1 à 6, dans laquelle l'acide non polymérique (c) ayant deux pKa ou plus ou le ou les sel(s) de celui-ci est un acide organique ou un ou plusieurs sel(s) de celui-ci, de préférence un acide organique hydrophile ou hydrosoluble ou un ou plusieurs sel(s) de celui-ci, et plus préférablement un acide phytique ou des sels de celui-ci.

8. Composition selon l'une quelconque des revendications 1 à 7, dans laquelle la quantité de l'acide non polymérique (c) ayant au moins deux valeurs de pKa ou le ou les sel(s) de celui-ci dans la composition est de 0,01 % à 15 % en poids, préférablement de 0,05 % à 10 % en poids, et plus préférablement de 0,1 % à 5 % en poids, par rapport au poids total de la composition.

9. Composition selon l'une quelconque des revendications 1 à 8, dans laquelle le polymère superabsorbant (d) est choisi parmi les polymères (méth)acryliques ou de (méth)acrylate réticulés, les amidons greffés avec un ou plusieurs polymère(s) acrylique(s) ou d'acrylate, et leurs mélanges.

10. Composition selon l'une quelconque des revendications 1 à 9, dans laquelle la quantité du polymère superabsorbant (d) dans la composition est de 0,01 % à 15 % en poids, préférablement de 0,05 % à 10 % en poids, et plus préférablement de 0,1 % à 5 % en poids, par rapport au poids total de la composition.

11. Composition selon l'une quelconque des revendications 1 à 10, dans laquelle la quantité de l'eau (e) présente dans la composition est de 50 % à 95 % en poids, préférablement de 60 % à 90 % en poids, et plus préférablement de 70 % à 85 % en poids, par rapport au poids total de la composition.

12. Composition selon l'une quelconque des revendications 1 à 11, le pH de la composition allant de 3 à 9, préférablement de 3,3 à 8,0 et plus préférablement de 3,5 à 8.

13. Composition selon l'une quelconque des revendications 1 à 12, dans laquelle la composition comprend en outre (f) au moins une huile.

14. Composition selon l'une quelconque des revendications 1 à 13, dans laquelle la composition est une composition cosmétique, préférablement une composition cosmétique cutanée, et plus préférablement une composition cosmétique de soins de la peau.

15. Procédé cosmétique pour une substance de kératine telle que la peau, comprenant
l'application sur la substance de kératine de la composition selon l'une quelconque des revendications 1 à 14 ; et
le séchage de la composition pour former un film cosmétique sur la substance de kératine.
